(19) Europäisches Patentamt
European Patent Office
Office européen des brevets

(11) **EP 2 909 159 B1**

(12) **EUROPÄISCHE PATENTSCHRIFT**

(45) Veröffentlichungstag und Bekanntmachung des Hinweises auf die Patenterteilung:
**21.09.2016 Patentblatt 2016/38**

(21) Anmeldenummer: **13776829.7**

(22) Anmeldetag: **16.10.2013**

(51) Int Cl.:
*C07C 5/10* (2006.01)      *C07C 5/29* (2006.01)
*C07C 13/18* (2006.01)

(86) Internationale Anmeldenummer:
**PCT/EP2013/071601**

(87) Internationale Veröffentlichungsnummer:
**WO 2014/060460 (24.04.2014 Gazette 2014/17)**

(54) **VERFAHREN ZUR HERSTELLUNG VON CYCLOHEXAN MIT AUS EINEM STEAMCRACKVERFAHREN STAMMENDEN AUSGANGSMATERIALIEN**

METHOD FOR PRODUCING CYCLOHEXANE BY USING STARTING MATERIALS ORIGINATING FROM A STEAM CRACKING PROCESS

PROCÉDÉ DE PRODUCTION DE CYCLOHEXANE EN UTILISANT DES MATÉRIAUX DE DÉPART ISSUS D'UN PROCÉDÉ DE VAPOCRAQUAGE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.10.2012 EP 12188949**

(43) Veröffentlichungstag der Anmeldung:
**26.08.2015 Patentblatt 2015/35**

(73) Patentinhaber: **BASF SE**
**67056 Ludwigshafen am Rhein (DE)**

(72) Erfinder:
• **TSCHIRSCHWITZ, Steffen**
**68199 Mannheim (DE)**
• **WISSEL-STOLL, Kathrin**
**67056 Ludwigshafen (DE)**
• **BÜRKLE, Jochen**
**68163 Mannheim (DE)**
• **KOSTOVA, Albena**
**68163 Mannheim (DE)**
• **SCHMITT, Markus**
**69115 Heidelberg (DE)**
• **WLOKA, Veronika**
**68163 Mannheim (DE)**
• **DEUERLEIN, Stephan**
**67061 Ludwigshafen (DE)**

• **BOSCH, Marco**
**68623 Lampertheim (DE)**
• **OEHLENSCHLÄGER, Steffen**
**67098 Bad Dürkheim (DE)**
• **SCHREIBER, Michael**
**68167 Mannheim (DE)**
• **AVERLANT, Gauthier Luc Maurice**
**60323 Frankfurt (DE)**
• **JONI, Joni**
**65843 Sulzbach (DE)**
• **PROCHAZKA, Roman**
**68163 Mannheim (DE)**
• **BOCK, Martin**
**67063 Ludwigshafen (DE)**
• **KINDLER, Alois**
**67269 Grünstadt (DE)**
• **MALKOWSKY, Daniela**
**67346 Speyer (DE)**
• **SPUHL, Katharina**
**B-1190 Forest (BE)**
• **BITTERLICH, Stefan**
**67246 Dirmstein (DE)**
• **PFEIFFER, Daniel**
**67435 Neustadt (DE)**

(56) Entgegenhaltungen:
**WO-A1-2011/069929      WO-A1-2011/069957
US-A- 3 311 667**

EP 2 909 159 B1

**Beschreibung**

Die vorliegende Erfindung betrifft ein Verfahren zur Herstellung von Cyclohexan durch Isomerisierung eines Kohlenwasserstoffgemisches (KG1) enthaltend Methylcyclopentan (MCP) in Gegenwart eines Katalysators. Der Katalysator ist vorzugsweise eine saure ionische Flüssigkeit. Als Ausgangsmaterial wird ein Strom (S1) eingesetzt, der aus einem Steamcrackverfahren stammt. Aus diesem Strom (S1) wird in einer Vorrichtung zur Aromatenabtrennung, bevorzugt Benzolabtrennung, das Kohlenwasserstoffgemisch (KG1) gewonnen, das gegenüber dem Strom (S1) einen reduzierten Aromatenanteil aufweist, gegebenenfalls kann (KG1) auch (nahezu) frei von Aromaten sein. In Abhängigkeit von der Art und Menge der in dem Kohlenwasserstoffgemisch (KG1) verbliebenen Aromaten, insbesondere bei der Anwesenheit von Benzol, kann vor der Isomerisierung zusätzlich eine Hydrierung von (KG1) durchgeführt werden. Darüber hinaus können in Abhängigkeit von der Anwesenheit sonstiger Komponenten von (KG1) gegebenenfalls weitere Aufreinigungsschritte vor oder nach der Isomerisierung bzw. Hydrierung durchgeführt werden. Aus dem bei der Isomerisierung anfallenden Kohlenwasserstoffgemisch (KG2) wird vorzugsweise hochreines (spezifikationsgerechtes) Cyclohexan isoliert, wobei die Spezifikationen beispielsweise durch die Verwendung des Cyclohexans für die dem Fachmann bekannte Herstellung von Caprolactam gegeben sind.

Cyclohexan ist ein wichtiges Wertprodukt der chemischen Industrie, das vorzugsweise durch Hydrierung von in weitgehend reiner Form bereitgestelltem Benzol hergestellt wird. Dabei stellt sich jedoch das Problem, dass das Benzol ein knappes Produkt ist und dass daher mit der Hydrierung zu Cyclohexan andere Verwendungen wie z.B. die Herstellung von Styrol konkurrieren. Daher besteht ein Anreiz, ein Herstellungsverfahren für Cyclohexan zu finden, dass von einem anderen Einsatzstoff als Rein-Benzol ausgeht.

Unter einem Steamcrackverfahren (das auch als Steamcracking-Verfahren bezeichnet wird) versteht man allgemein ein seit langem bekanntes petrochemisches Verfahren, bei dem hauptsächlich längerkettige Kohlenwasserstoffe in Gegenwart von Wasserdampf durch thermisches Cracken in kurzkettige Kohlenwasserstoffe umgewandelt werden. Die dabei als Ausgangsmaterial eingesetzten längerkettigen Kohlenwasserstoffe sind insbesondere in der Erdölraffinerie anfallendes Naphtha, darüber hinaus können aber auch leichte Kohlenwasserstofffraktionen aus Begleitgasen der Ölförderung oder abgetrennte schwerere Fraktionen ("C2+") aus Erdgas verwendet werden. Das im Rahmen der vorliegenden Erfindung verwendete Kohlenwasserstoffgemisch (KG1) stammt bevorzugt aus einem Steamcrackverfahren, bei dem überwiegend Naphtha als Einsatzstoff verwendet wird ("Naphthacracker"). Als Produkte entstehen bei einem Steamcrackverfahren vor allem Wasserstoff, Methan, kurzkettige Kohlenwasserstoffe wie Ethen, Propen und Butene, sowie aromatenhaltiges Pyrolysebenzin (umfasst unter anderem Benzol und Toluol sowie sonstige Kohlenwasserstoffe, beispielsweise lineare, verzweigte oder zyklische $C_5$-$C_6$-Alkane wie MCP).

Das bei einem Steamcrackverfahren anfallende Pyrolysebenzin wird in der Regel, gegebenenfalls nach einer Leichtsiederabtrennung und/oder der Abtrennung einer an C9-Kohlenwasserstoffen angereicherten Fraktion, einer destillativen Auftrennung in verschiedene Fraktionen, bevorzugt jeweils eine an Benzol, eine an Toluol- und eine an Xylolen angereicherte Fraktion unterzogen. Diese Sequenz wird gegebenenfalls ergänzt durch eine oder mehrere Selektivhydrierungen zwecks Umsetzung von Olefinen und/oder Dienen und/oder olefinischen Seitengruppen von Aromaten und/oder Schwefelverbindungen.

Weiterhin ist es bekannt, dass Cyclohexan nicht nur, wie vorstehend beschrieben, durch Hydrierung von Benzol sondern auch durch Isomerisierung von MCP hergestellt werden kann. Als Katalysatoren für eine derartige Isomerisierung werden bevorzugt saure Katalysatoren im Sinne einer Lewis- oder Brönstedt-Säure eingesetzt wie z.B. Friedel-Crafts-Katalysatoren oder auch saure ionische Flüssigkeiten.

US-A 2003/0109767 offenbart ein Verfahren zur Isomerisierung von $C_5$-$C_8$-Paraffinkohlenwasserstoffen (Paraffinen) in Gegenwart einer ionischen Flüssigkeit als Katalysator. Die ionische Flüssigkeit umfasst als Kationen stickstoffhaltige Heterocyclen oder stickstoffhaltige Aliphate, die entsprechenden Anionen sind von Metallhalogeniden abgeleitet. Bei den zu isomerisierenden Paraffinen handelt es sich um lineare Alkane wie n-Hexan oder n-Octan sowie monosubstituierte Alkane wie 3-Methylhexan bzw. Mischungen davon. Durch das in US-A 2003/0109767 beschriebene Verfahren sollen Paraffine mit einem höheren Verzweigungsgrad hergestellt werden. Im Gegensatz dazu hat beispielsweise Cyclohexan gegenüber MCP einen geringeren Verzweigungsgrad. Weiterhin sind in US-A 2003/0109767 keine Angaben dahingehend enthalten, woher die zur Isomerisierung eingesetzten Kohlenwasserstoffe stammen oder dass mit dem Ausgangsgemisch eine Aromatenabtrennung durchgeführt wird bzw. etwaig verbliebene Aromaten vor der Isomerisierung gegebenenfalls hydriert werden.

In dem in EP-A 1 403 236 beschriebenen Isomerisierungsverfahren soll ebenfalls ein höherer Verzweigungsgrad in den in Gegenwart einer ionischer Flüssigkeit zu isomerisierenden Paraffinen (Kohlenwasserstoffen) erhalten werden. Das Isomerisierungsverfahren wird zudem in Gegenwart von zyklischen Kohlenwasserstoffen als Additiven und in einem Reaktionsmedium durchgeführt, wobei die zyklischen Kohlenwasserstoffe ein tertiäres Kohlenstoffatom als Struktureinheit enthalten bzw. durch das Reaktionsmedium in eine entsprechende Verbindung mit einer solchen Struktureinheit überführt werden. Vorzugsweise werden Methylcyclohexan oder Dimethylcyclopentan als solche zyklischen Kohlenwasserstoffadditive eingesetzt. Als zu isomerisierende Paraffine werden lineare Alkane wie n-Butan oder n-Octan sowie monomethylsubstituierte Alkane wie 2-Methylhexan eingesetzt. Die ionischen Flüssigkeiten basieren vorzugsweise auf stickstoffhaltigen Heterocyclen oder stickstoffhaltigen Aliphaten als Kationen sowie auf anorganischen Anionen wie Aluminiumhalogeniden. In EP-A 1 403 236 sind ebenfalls keine Angaben enthalten, woher die zur Isomerisierung eingesetzten Kohlenwasserstoffe stammen oder dass mit dem Ausgangsgemisch eine Aromatenabtrennung durchgeführt wird bzw. etwaig verbliebene Aromaten vor der Isomerisierung gegebenenfalls hydriert werden.

US-A 2005/0082201 offenbart ein Verfahren zur Herstellung von Benzin mit einem niedrigen Benzolgehalt, wobei zunächst in einem ersten Verfahrensschritt ein Kohlenwasserstoffgemisch, das Benzol, Olefine und schwefelhaltige Verbindungen wie Thiophene enthält, in eine Destillationskolonne eingespeist wird, aus der über Kopf die leichtsiedenden Verbindungen, über einen Seitenabzug eine benzolhaltige Fraktion und aus dem Kolonnensumpf die Schwersieder abgetrennt werden. In einer zweiten Verfahrensstufe wird die aus dem Seitenabzug gewonnene Fraktion in Gegenwart eines Hydrierkatalysators hydriert, wobei Benzol zu Cyclohexan und die Thiophene in Schwefelwasserstoff überführt werden. Das bei der zweiten Verfahrensstufe anfallende cyclohexanhaltige Gemisch eignet sich zur Herstellung von Benzin mit einem niedrigen Benzolgehalt. Eine Isolierung des darin enthaltenen Cyclohexans oder eine Isomerisierung generell, beispielsweise von MCP zu Cyclohexan, sind in US-A 2005/0082201 nicht offenbart.

WO 2010/027987 betrifft ein weiteres Verfahren zur Verringerung der Konzentration an Benzol in einem kohlenwasserstoffhaltigen Gemisch. In einer ersten Trennstufe wird eine benzolhaltige Fraktion, die Benzol und andere $C_6$-Kohlenwasserstoffe umfasst, von einer Schwersiederfraktion abgetrennt, die Kohlenstoffe mit sieben und mehr Kohlenstoffatomen umfasst. Die benzolhaltige Fraktion wird anschließend hydriert unter Erhalt einer Kohlenwasserstofffraktion mit einem reduzierten Benzolgehalt. Bei der Hydrierung von Benzol wird Cyclohexan gebildet. Auch in WO 2010/027987 sind keine Hinweise enthalten, dass aus dem bei der Hydrierung erhaltenen Gemisch Cyclohexan isoliert werden kann, vielmehr soll auch dieses Verfahrensprodukt zur Benzinherstellung verwendet werden. Ebenso wenig offenbart dieses Dokument eine Isomerisierung, beispielsweise von MCP zu Cyclohexan.

US-A 3,311,667 betrifft ein Verfahren zur Entfernung von Benzol aus einem Gemisch, das nachfolgend in eine Isomerisierung von MCP zu Cyclohexan eingespeist wird. Bei der Hydrierung wird Benzol in Gegenwart eines geeigneten Katalysators, beispielsweise einem Metallkatalysator auf Kieselgur, mit Wasserstoff zu Cyclohexan hydriert. Die Isomerisierung von MCP zu Cyclohexan wird in Gegenwart von Metallhalogeniden wie säureverstärktem Aluminiumhalid, durchgeführt. In US-A 3,311,667 ist jedoch nicht beschrieben, woher die zur Isomerisierung eingesetzten Kohlenwasserstoffe stammen oder dass mit dem Ausgangsgemisch eine Aromatenabtrennung durchgeführt wird. Ebenso wenig ist dort offenbart, dass zur Isomerisierung auch eine saure ionische Flüssigkeit eingesetzt werden kann.

EP-A 1 995 297 offenbart ein Verfahren sowie eine zugehörige Vorrichtung zur Hydrierung und Decyclisierung von Benzol und der Isomerisierung von $C_5$-$C_6$-Paraffinen, die in einem Gemisch enthalten sind, das höchstens 1 Gew.-% Benzol enthält. Zur Hydrierung von Benzol können metallhaltige Katalysatoren verwendet werden, wobei sich als Metall die Elemente der Platingruppe, Zinn oder Kobalt und Molybdän eignen. Zur Isomerisierung des bei der Hydrierung erhaltenen Gemisches, das eine Restmenge an Benzol enthalten kann, werden insbesondere Zeolithe als Katalysator eingesetzt. Bei dem in EP-A 1 995 297 beschriebenen Verfahren werden bei der Isomerisierung die Parameter so eingestellt, dass eine Öffnung der bei der Benzolhydrierung erhaltenen Cyclohexanringe zu Isoalkanen erzielt wird. Bei diesem Verfahren geht es also nicht vordergründig um die Herstellung von Cyclohexan, sondern um die Herstellung von Alkanen mit einem hohen Verzweigungsgrad. Darüber hinaus sind auch in EP-A 1 995 297 keine Angaben enthalten, dass zur Isomerisierung auch eine saure ionische Flüssigkeit eingesetzt werden kann bzw. woher die zur Isomerisierung eingesetzten Kohlenwasserstoffe stammen oder dass mit dem Ausgangsgemisch eine Aromatenabtrennung durchgeführt wird. Ein sinngemäßes Verfahren zu EP-A 1 995 297 wird in EP-A 1 992 673 beschrieben.

US-A 2,846,485 offenbart ein Verfahren zur Herstellung von hochreinem Cyclohexan und Benzol, wobei ein Gemisch eingesetzt wird, das n-Hexan, Benzol, MCP, Cyclohexan und Dimethylpentane (DMP) enthält. In einer ersten Extraktivdestillationszone wird Benzol von den übrigen Eduktkomponenten abgetrennt. Das von Benzol weitgehend befreite Edukt wird mit einem Gemisch vereinigt, das Cyclohexan und MCP enthält und aus dem Sumpf einer zweiten fraktionierenden Destillationszone stammt. Das so vereinigte Gemisch wird in eine erste fraktionierende Destillationszone eingespeist, wobei über Kopf eine MCP-haltige Fraktion und aus dem Sumpf eine cyclohexanhaltige Fraktion abgetrennt wird.

Das Überkopfprodukt der ersten fraktionierenden Destillationszone wird zunächst in eine Isomerisierungszone geführt, in der die Hauptmenge an MCP zu Cyclohexan unter Verwendung von Friedel-Crafts-Katalysatoren wie Aluminiumchlorid, das zusätzlich HCl enthalten kann, isomerisiert wird. Das Isomerisierungsprodukt wird in die vorstehend beschriebene zweite fraktionierende Destillationszone eingeleitet, um dort n-Hexan und Leichtsieder als Kopfprodukt abzutrennen. Das Sumpfprodukt aus der ersten fraktionierenden Destillationszone wird in eine zweite Extraktivdestillationszone überführt, in der aus dem Sumpf ein Cyclohexan enthaltendes Gemisch von dem über Kopf abgezogenen DMP abgetrennt wird.

Das in US-A 2,846,485 beschriebene Verfahren ist nachteilig, da es (unter anderem) apparativ sehr aufwändig ist. Die Abtrennung des eigentlichen Verfahrensproduktes Cyclohexan von DMP erfolgt erst zum Schluss des Verfahrens, da das bei der Isomerisierung von MCP gebildete Cyclohexan in eine DMP-haltige Fraktion rückgeleitet wird. Weiterhin sind in US-A 2,846,485 keine Angaben dahingehend enthalten, woher die zur Isomerisierung eingesetzten Kohlenwasserstoffe stammen oder dass im Ausgangsgemisch enthaltene Aromaten vor der Isomerisierung gegebenenfalls hydriert werden.

Ionische Flüssigkeiten eignen sich unter anderem als Katalysatoren für die Isomerisierung von Kohlenwasserstoffen. Eine entsprechende Verwendung einer ionischen Flüssigkeit ist beispielsweise in WO 2011/069929 offenbart, wo eine spezielle Auswahl von ionischen Flüssigkeiten in Gegenwart eines Olefins zur Isomerisierung von gesättigten Kohlenwasserstoffen eingesetzt wird, insbesondere zur Isomerisierung von Methylcyclopentan (MCP) zu Cyclohexan. Ein sinngemäßes Verfahren ist in WO 2011/069957 beschrieben, allerdings erfolgt dort die Isomerisierung nicht in Gegenwart eines Olefins, sondern mit einer Kupfer (II)-Verbindung.

Die der vorliegenden Erfindung zugrunde liegende Aufgabe besteht in der Bereitstellung eines neuen Verfahrens zur Herstellung von Cyclohexan aus Ausgangsmaterialien, die aus einem Steamcrackverfahren stammen. Diese Ausgangsmaterialien enthalten immer MCP, ansonsten können sie hinsichtlich ihrer sonstigen Komponenten variieren. Weiterhin soll die Möglichkeit bestehen, dass gegebenenfalls in den Ausgangsmaterialien enthaltenes Cyclohexan zurückgewonnen werden kann.

Gelöst wird die Aufgabe durch ein Verfahren zur Herstellung von Cyclohexan umfassend die folgenden Schritte

a) Isomerisierung eines Kohlenwasserstoffgemisches (KG1) enthaltend Methylcyclopentan (MCP) in Gegenwart eines Katalysators unter Erhalt eines Kohlenwasserstoffgemisches (KG2) enthaltend Cyclohexan, wobei (KG1) in einer Vorrichtung zur Aromatenabtrennung, bevorzugt zur Benzolabtrennung, die einem Steamcrackverfahren nachgeschaltet ist, aus einem aus dem Steamcrackverfahren stammenden Strom (S1) gewonnen wird, und

b) Isolierung von Cyclohexan aus dem Kohlenwasserstoffgemisch (KG2).

Durch das erfindungsgemäße Verfahren kann in vorteilhafter Weise reines, insbesondere hochreines (spezifikationsgerechtes) Cyclohexan hergestellt werden, wobei die Spezifikationen beispielweise durch die Verwendung des Cyclohexans für die dem Fachmann bekannte Herstellung von Caprolactam gegeben sind. Das erfindungsgemäße Verfahren ist vorteilhaft hinsichtlich des apparativen Aufwandes, weiterhin können hohe Ausbeuten an Cyclohexan erhalten werden.

Ein weiterer wichtiger Vorteil des erfindungsgemäßen Verfahrens ist darin zu sehen, dass es sehr flexibel durchgeführt werden kann. Je nach Zusammensetzung des eingesetzten Kohlenwasserstoffgemisches (KG1) können vor der Isomerisierung gemäß Schritt a) unterschiedliche Zwischenschritte (beispielsweise Aufreinigungsschritte) einzeln oder in beliebigen Kombinationen durchgeführt werden. Sofern in (KG1) Schwersieder, insbesondere DMP, enthalten sind, können diese vor der Isomerisierung und gegebenenfalls auch vor der Hydrierung vollständig oder zumindest weitgehend aus (KG1) abgetrennt werden ("Schwersieder- bzw. DMP-Vorabtrennung"). Weiterhin ist die Durchführung einer Hydrierung nur erforderlich, sofern (KG1) Aromaten, insbesondere Benzol und gegebenenfalls Cyclohexen, (im nennenswerten Umfang) enthält. Sofern in (KG1) Leichtsieder enthalten sind, können diese ebenfalls an geeigneter Stelle vor der Isomerisierung entfernt werden.

Die spezifischen (zusätzlichen) Vorteile, die mit den vorstehenden Verfahrensvarianten (Ausführungsformen) der vorliegenden Erfindung verbunden sind, werden nachfolgend nochmals verdeutlicht. Sofern einzelne Ausführungsformen miteinander kombiniert werden, ergänzen sich die mit den jeweiligen Ausführungsformen verbundenen Vorteile entsprechend. An dieser Stelle wird bereits darauf hingewiesen, dass trotz der zusätzlichen Anwesenheit von beispielsweise DMP, Benzol und/oder Leichtsiedern im Kohlenwasserstoffgemisch (KG1) das erfindungsgemäße Verfahren auch in seiner Grundform (also nur mit den Schritten a) und b)) durchgeführt werden kann, ohne dass zusätzlich eine DMP-Vorabtrennung, eine Hydrierung und/oder eine Leichtsieder-Abtrennung zusätzlich durchgeführt werden. bei dieser Fallkonstellation treten die spezifischen (zusätzlichen) Vorteile, die mit den konkreten Verfahrensvarianten verbunden sind, nicht zusätzlich auf.

In einigen Ausführungsformen der vorliegenden Erfindung können im eingesetzten Kohlenwasserstoffgemisch (KG1) auch Schwersieder, insbesondere DMP, enthalten sein. Sofern DMP vor dem eigentlichen Cyclohexan-Herstellungsprozess abgetrennt wird, kann die äußerst aufwändige Trennung, insbesondere Destillation, von DMP aus dem Verfahrensprodukt Cyclohexan umgangen werden, insbesondere, wenn es sich bei dem DMP um 2,4-Dimethylpentan (2,4-DMP) handelt und dieses im Ausgangsgemisch in einer Konzentrationen > 100 ppm vorliegt. Dadurch wird der energetische und apparative Aufwand bei der Herstellung von reinem bzw. hochreinem Cyclohexan deutlich reduziert. Die Abtrennung der DMP vom eigentlichen Verfahrensprodukt Cyclohexan ist nämlich technisch ziemlich anspruchsvoll und aufwändig, insbesondere wenn es sich um das DMP-Isomer 2,4-Dimethylpentan (2,4-DMP) handelt. Der Normalsiedepunkt von 2,4-DMP ist mit 80,52 °C dem Normalsiedepunkt von Cyclohexan (80,78 °C) sehr ähnlich, die Normalsiedepunkte der anderen DMP-Isomere weisen hingegen einen größeren Abstand zu Cyclohexan auf (2,3-DMP hat beispielsweise einen Normalsiedepunkt von 89,88 °C).

Durch das erfindungsgemäße Verfahren kann bei dieser Verfahrensvariante in vorteilhafter Weise das in (KG1) enthaltene DMP vollständig oder nahezu vollständig durch die Vorabtrennung aus (KG1) entfernt werden. Besonders bevorzugt wird das erfindungsgemäße Verfahren so durchgeführt, dass das in (KG1) enthaltene DMP vollständig oder nahezu vollständig (bis zu 2 % bezogen auf die im Ausgangsgemisch enthaltenen Menge aller DMP-Isomere) per DMP-Vorabtrennung aus (KG1) abgetrennt wird.

Das erfindungsgemäße Verfahren kann weiterhin unabhängig davon durchgeführt werden, ob im eingesetzten Kohlenwasserstoffgemisch bereits Cyclohexan enthalten ist oder nicht. Sofern in den eingesetzten Kohlenwasserstoffgemischen neben DMP auch Cyclohexan selber enthalten ist, wird dieses in (KG1) enthaltene Cyclohexan im Rahmen des erfindungsmäßen Verfahrens bevorzugt zusammen mit DMP über Sumpf abgetrennt. Der mit dieser Fallkonstellation verbundene Nachteil einer Verringerung der Cyclohexan-Produktmenge wird durch die vorstehend beschriebene Verringerung des energetischen und apparativen Aufwands jedoch überkompensiert.

In einer Ausführungsform der vorliegenden Erfindung kann dieses in (KG1) enthaltene Cyclohexan jedoch wieder zurückgewonnen werden. Bei dieser Ausführungsform wird das gemeinsam mit den Schwersiedern, insbesondere mit DMP, aus dem Verfahren ausgeschleuste Cyclohexan destillativ, vorzugsweise durch eine Extraktiv- oder Schleppmitteldestillation, von DMP wieder abgetrennt. Das dabei erhaltene Cyclohexan, das im Wesentlichen frei von DMP ist, kann dem eigentlichen Verfahrensprodukt (Cyclohexan, das nach dem erfindungsgemäßen Verfahren hergestellt wird) wieder zugeführt oder an einer sonstigen Stelle in das erfindungsgemäße Verfahren eingespeist werden. Der Vorteil bei dieser Verfahrensvariante gegenüber einer Abtrennung aus einer Stelle weiter hinten im Verfahren (stromabwärts), also z. B. aus dem Cyclohexan-Produktstrom, ist darin zu sehen, dass die DMP-Abtrennung von einer deutlich kleineren Cyclohexanmenge durchzuführen ist, da DMP nur von dem im Kohlenwasserstoff-Ausgangsgemisch enthaltenen Cyclohexan abgetrennt wird und nicht auch von dem bei der Hydrierung und/oder Isomerisierung gebildeten Cyclohexan, das das eigentliche Verfahrensprodukt darstellt. Demzufolge sind für diese separate DMP-/Cyclohexan-Trennung kleinere Apparaturen und eine geringere Energiemenge erforderlich.

In einigen Ausführungsformen der vorliegenden Erfindung können trotz der dem Steamcrackverfahren nachgeschalteten Aromatenabtrennung im eingesetzten Kohlenwasserstoffgemisch (KG1) auch noch Aromaten, insbesondere Benzol, enthalten sein. Aufgrund der bei diesen Ausführungsformen durchgeführten Hydrierung der Aromaten kann die Isomerisierung gemäß Schritt a), vorzugsweise in Gegenwart einer sauren ionischen Flüssigkeit, in vorteilhafter Weise durchgeführt werden. Der Vorteil ist darin zu sehen, dass die in (KG1) verbliebenen Aromaten, insbesondere Benzol, durch eine vorgeschaltete Hydrierung vollständig oder zumindest weitgehend abgetrennt in die entsprechenden gesättigten Kohlenwasserstoffe umgewandelt werden können. Demzufolge wird die andernfalls eintretende Desaktivierung der zur Isomerisierung, insbesondere zur Isomerisierung von MCP zu Cyclohexan, eingesetzten Katalysatoren, insbesondere von sauren ionischen Flüssigkeiten, durch Aromaten, insbesondere durch Benzol oder andere ungesättigte Verbindungen verringert oder ganz vermieden.

Weiterhin hat die Hydrierung der in (KG1) enthaltenen Aromaten, sofern diese Benzol umfassen, den Vorteil, dass die Menge des anfallenden Produkts um das bei der Hydrierung des Benzols anfallende Cyclohexan erhöht wird.

Die Entfernung der verbliebenen Aromaten, insbesondere von Benzol, hat den zusätzlichen Vorteil, dass gegebenenfalls nachfolgend ausgeführte destillative Aufarbeitungsschritte erleichtert werden, weil so die andernfalls auftretende Bildung von Azeotropen von Aromaten wie zum Beispiel Benzol mit gesättigten $C_6$-$C_7$-Alkanen vermieden wird.

In einigen Ausführungsformen der vorliegenden Erfindung können im eingesetzten Kohlenwasserstoffgemisch (KG1) auch Leichtsieder enthalten sein. Prinzipiell kann eine Abtrennung von Leichtsiedern, insbesondere von Isohexanen, an verschiedenen Stellen im Verfahren erfolgen. Besonders vorteilhaft ist es jedoch, insbesondere im Fall von Aromaten-haltigen, insbesondere Benzol-haltigen, Kohlenwasserstoffgemischen (KG1), die Abtrennung von Leichtsiedern nach der Hydrierung und vor der Isomerisierung durchzuführen. Eine Abtrennung von Leichtsiedern vor der Hydrierung hätte nämlich den Nachteil, dass das vor der Hydrierung im Kohlenwasserstoffgemisch enthaltene Benzol mit mindestens einem Teil der abzutrennenden Leichtsiedern Azeotrope bildet und daher zumindest teilweise zusammen mit den Leichtsiedern abgetrennt werden würde. Dadurch würde sich die Produktmenge um die zusammen mit den Leichtsiedern abgetrennte Menge an Benzol verringern.

Eine Abtrennung von Leichtsiedern nach der Isomerisierung hätte wiederum den Nachteil, dass die Leichtsieder die zu isomerisierenden Kohlenwasserstoffe, insbesondere MCP, verdünnen und so zu einer Verringerung der Raum-Zeit-Ausbeute in der Isomerisierung führen würden. Zudem ist die Abtrennung von Isohexanen vor der Isomerisierung vorteilhaft, weil so die Triebkraft für die Isomerisierung von n-Hexan zu Isohexanen in der nachfolgenden Isomerisierungsstufe erhöht wird. Die Isomerisierung von n-Hexan zu Isohexanen in der Isomerisierungsstufe ist wiederum bedeutsam, weil aufgrund der Lage der Siedepunkte n-Hexan (Normalsiedepunkt 68,7 °C) deutlich schwieriger als die Isohexane (Normalsiedepunkte 49,7 bis 63,3 °C) von MCP (Normalsiedepunkt 71,7 °C) abzutrennen ist. Da sich aber bevorzugt an die Isomerisierungsstufe eine destillative Trennung anschließt, in der MCP zusammen mit offenkettigen Hexanen vom gebildeten Cyclohexan abgetrennt und vor bzw. in die Isomerisierung zurückgeführt werden, was wiederum die Ausschleusung der offenkettigen Hexane aus dem Verfahren nötig macht, ist es aufgrund der genannten Lage der Siedepunkte vorteilhaft, die offenkettigen Hexane überwiegend in Form von Isohexanen aus dem Verfahren auszuschleusen, während eine durch die Isomerisierung von n-Hexan begrenzte Aufpegelung desselben in Kauf genommen werden kann.

Im Rahmen der vorliegenden Erfindung kann eine Destillation in den dem Fachmann bekannten Ausführungsformen (siehe z B. Kirk-Othmer Encyclopedia of Chemical Technology Published Online: 17 AUG 2001, Vol. 8 S. 739 ff.) durchgeführt werden. Die jeweiligen Destillationstechniken werden in den entsprechenden dem Fachmann bekannten Vorrichtungen durchgeführt. Die Durchführung einer Extraktivdestillation zur Auftrennung engsiedender Substanzen ist beispielsweise in US A 4,053,369, US-A 4,955,468 oder WO 02/22528 beschrieben. Die Destillation unter Verwendung von Trennwandkolonnen ist beispielsweise in EP1127601 B1 beschrieben.

Im Rahmen der vorliegenden Erfindung wird eine Destillation vorzugsweise als Rektifikation ausgestaltet, das heißt, unter dem Begriff "Destillationskolonne" ist vorzugsweise eine Rektifizierkolonne zu verstehen. Unter "Rektifikation", die in einer entsprechenden Rektifizierkolonne (Rektifiziervorrichtung), auch Rektifikationskolonne oder Rektifikationsvorrichtung genannt, durchgeführt wird, wird Folgendes verstanden: Bei der Rektifikation wird der durch Destillation erzeugte Dampf in einer Rektifizierkolonne im Gegenstrom zu einem Teil seines Kondensates geführt. Auf diese Weise werden leichter flüchtige Komponenten im Kopf- und schwerer flüchtige im Sumpfprodukt der Rektifizierkolonne angereichert.

Im vorliegenden Zusammenhang schließt der Begriff "Rektifikationskolonne" jeweils dem Fachmann bekannte Nebenapparate wie z. B. einen oder mehrere Sumpfverdampfer, mindestens einen Kondensator sowie gegebenenfalls Behälter und Pumpen, mit ein. Dementsprechend ist die Entnahme von Strömen aus der Rektifikationskolonne so zu verstehen, dass der jeweilige Strom gegebenenfalls über einen oder mehrere dieser Nebenapparate geleitet wird, gegebenenfalls auch unter Änderung des Aggregatzustands und/oder Rückleitung eines Teils des entnommenen Stroms. So ist zum Beispiel die Entnahme eines Stroms über den Kopf der Rektifikationskolonne so zu verstehen, dass der am Kopf der Kolonne anfallende Brüdenstrom mindestens teilweise kondensiert wird und nachfolgend in einen Rücklaufstrom und einen Kopfproduktstrom aufgeteilt wird. Der Kopfproduktstrom ist dann gleichzusetzen mit dem im nachfolgenden Text vereinfachend als über Kopf entnommenen Strom bezeichneten Strom. Analog schließt auch die Nennung der Zuführung eines Stromes zu einer Rektifikationskolonne die Option ein, dass der betreffende Strom vor dem Eintritt in die Kolonne selbst einen oder mehrere Nebenapparate durchläuft wie beispielsweise einen Vorwärmer oder Vorverdampfer.

Im Rahmen der vorliegenden Erfindung werden unter dem Begriff "Dimethylpentane" (DMP) alle bekannten Isomere von Dimethylpentan verstanden, insbesondere 2,2-Dimethylpentan (2,2-DMP; Normalsiedepunkt: 79,17 °C), 2,3-Dimethylpentan (2,3-DMP; Normalsiedepunkt: 89,88 °C), 3,3-Dimethylpentan (3,3-DMP; Normalsiedepunkt: 86,09 °C) und 2,4-Dimethylpentan (2,4-DMP; Normalsiedepunkt: 80,52 °C). Dies bedeutet, dass in den Ausführungsformen des erfindungsgemäßen Verfahrens, die DMP-haltige Gemische bzw. Ströme betreffen, in den entsprechenden Gemischen bzw. Strömen mindestens ein Dimethylpentan-Isomer enthalten ist, vorzugsweise handelt es sich um Gemische von zwei oder mehr Dimethylpentan-Isomeren, wobei eines dieser Isomere vorzugsweise 2,4-Dimethylpentan ist.

Im Rahmen der vorliegenden Erfindung werden unter dem Begriff "Verbindungen mit einem Normalsiedepunkt von 79 bis 84 °C" alle Kohlenwasserstoffe verstanden, die bei Normaldruck im Bereich von 79 bis 84 °C sieden und die einzeln oder als Gemisch im erfindungsgemäßen Verfahren zunächst im Kohlenwasserstoffgemisch (KG1) enthalten sein können. Im Rahmen des erfindungsgemäßen Verfahrens können einzelne oder mehrere dieser Verbindungen voneinander abgetrennt werden. Gegebenenfalls können auch einzelne oder mehrere dieser Verbindungen im nachfolgenden Text separat als Bestandteil von Gemischen oder Strömen aufgeführt sein. Sofern dies der Fall ist, sind nur die jeweils konkret aufgeführten Verbindungen zwingender Bestandteil des entsprechenden Gemisches oder Stromes; die sonstigen Verbindungen mit einem Normalsiedepunkt von 79 bis 84 °C, die nicht namentlich im entsprechenden Strom oder Gemisch aufgeführt sind, können (sofern nicht anders aufgeführt oder beispielsweise infolge einer vorausgegangenen Abtrennung nicht mehr möglich) ebenfalls im entsprechenden Strom oder Gemisch vorhanden sein.

Gegebenenfalls können einzelne oder mehrere dieser Verbindungen auch unter die Definition einer anderen Auswahl von Verbindungen fallen, wie beispielsweise unter die Definition des Begriffs "$C_5$-$C_6$-Alkane".

Beispiele für Verbindungen mit einem Normalsiedepunkt von 79 bis 84 °C sind Cyclohexan (80,78 °C), 2,2-DMP (79,17 °C), 2,4-DMP (80,52 °C), 2,2,3-Trimethylbutan (80,87 °C) und Benzol (80,08 °C).

Sinngemäßes wie vorstehend für die Verbindungen mit einem Normalsiedepunkt von 79 bis 84 °C aufgeführt gilt im Rahmen der vorliegenden Erfindung auch für Verbindungen, die unter den Begriff "Schwersieder mit einem Normalsiedepunkt > 84 °C" fallen. Beispiele für Schwersieder mit einem Normalsiedepunkt > 84 °C sind 3,3-DMP (86,09 °C), 2,3-DMP (89,88 °C) und die iso-Heptane 2-Methylhexan (2-MH; 90,06 °C), 3-Methylhexan (3-MH; 91,87 °C) und 3-Ethylpentan (3-EP; 93,45 °C).

Im Rahmen der vorliegenden Erfindung können die beiden vorgenannten Gruppen von Verbindungen (Verbindungen mit einem Normalsiedepunkt von 79 bis 84 °C sowie Schwersieder mit einem Normalsiedepunkt > 84 °C) gegebenenfalls auch zu einer Gruppe von Verbindungen zusammengefasst sein. In dieser Fallkonstellation werden die Verbindungen entsprechend als "Schwersieder mit einem Normalsiedepunkt > 78 °C" bezeichnet. Die vorstehenden Ausführungen zu den beiden Einzelgruppen gelten sinngemäß auch für diese Gruppe von Verbindungen. Im Rahmen der vorliegenden Erfindung werden die Schwersieder mit einem Normalsiedepunkt > 78 °C auch als "Alkane mit 7 oder mehr Kohlenstoffatomen" bezeichnet bzw. die Alkane mit 7 oder mehr Kohlenstoffatomen stellen eine Untergruppe der Schwersieder mit einem Normalsiedepunkt > 78 °C dar.

Weiterhin kann im Rahmen der vorliegenden Erfindung die Gruppe der Verbindungen mit einem Normalsiedepunkt > 84 °C auch als Untergruppe in der Gruppe enthalten sein, die als "schwerer als Cyclohexan siedende Komponenten" bezeichnet wird. Die letztgenannte Gruppe umfasst also zusätzlich auch Verbindungen mit einem Normalsiedepunkt > 80,78 °C bis einschließlich 84 °C.

Im Rahmen der vorliegenden Erfindung bedeutet der Begriff "Großteil" im Zusammenhang mit einem Strom (Feedstrom) -soweit nicht anders ausgeführt mindestens 50 %, vorzugsweise mindestens 80 %, mehr bevorzugt mindestens 95 %, insbesondere mindestens 99 % (die Werte sind als Anteile am jeweiligen Feedstrom zu verstehen).

Nachfolgend wird das erfindungsgemäße Verfahren zur Herstellung von Cyclohexan mit aus einem Steamcrackverfahren stammenden Ausgangsmaterialien näher definiert. In diesem Zusammenhang wird auch auf die Figuren 1 bis 7 verwiesen. Figur 1 zeigt das erfindungsgemäße Verfahren in seiner Grundform unter Berücksichtigung der Schritte a) und b). In den Figuren 2 bis 5 sind spezielle Ausgestaltungen des erfindungsgemäßen Verfahrens illustriert, die dem Schritt a) vorgeschaltet sind. Figur 6 zeigt eine spezielle Ausgestaltung zur Rückgewinnung von Cyclohexan, sofern dieses zusammen mit Schwersiedern, insbesondere mit DMP, bereits im Kohlenwasserstoffgemisch (KG1) enthalten ist. Die Figur 7 betrifft eine spezielle Ausgestaltung der Isolierung von Cyclohexan gemäß Schritt b). Sämtliche Figuren werden im nachfolgenden Text an entsprechender Stelle näher definiert.

Im Rahmen der vorliegenden Erfindung erfolgt in Schritt a) die Isomerisierung eines Kohlenwasserstoffgemisches (KG1) enthaltend Methylcyclopentan (MCP), in Gegenwart eines Katalysators unter Erhalt eines Kohlenwasserstoffgemisches (KG2) enthaltend Cyclohexan, wobei (KG1) in einer Vorrichtung zur Aromatenabtrennung, bevorzugt zur Benzolabtrennung, die einem Steamcrackverfahren nachgeschaltet ist, aus einem aus dem Steamcrackverfahren stammenden Strom (S1) gewonnen wird.

Prinzipiell können im Rahmen der vorliegenden Erfindung beliebige Kohlenwasserstoffe als Kohlenwasserstoffgemisch (KG1) eingesetzt werden, unter der Voraussetzung, dass mindestens einer der eingesetzten Kohlenwasserstoffe MCP ist, das gemäß Schritt a) in Gegenwart eines Katalysators, insbesondere in Gegenwart einer sauren ionischen Flüssigkeit, einer Isomerisierung zu Cyclohexan unterzogen wird. Gegebenenfalls können neben MCP auch weitere in (KG1) enthaltene Kohlenwasserstoffe isomerisiert werden. Der Fachmann weiß aufgrund seines allgemeinen Fachwissens, welche Kohlenwasserstoffe durch Katalysatoren, insbesondere durch saure ionische Flüssigkeiten, isomerisierbar sind.

Vorzugsweise werden im Rahmen der vorliegenden Erfindung Kohlenwasserstoffgemische (KG1) eingesetzt, die außer MCP weitere Komponenten umfassen, beispielsweise Kohlenwasserstoffe die hydrierbar sind. Gegebenenfalls können in solchen Gemischen auch Verbindungen enthalten sein, die selber keine Kohlenwasserstoffe, jedoch mit diesen mischbar sind.

Die einzelnen Komponenten des Kohlenwasserstoffgemischs (KG1) können in beliebigen Konzentrationen/Verhältnissen untereinander vorliegen. Vorzugsweise enthält das Kohlenwasserstoffgemisch (KG1) zu mindestens 90 Gew.-%, bevorzugt mindestens 95 Gew.-% Kohlenwasserstoffe mit 5 bis 8 Kohlenstoffatomen, unter der Voraussetzung, dass mindestens einer der eingesetzten Kohlenwasserstoffe MCP ist. Die Kohlenwasserstoffe können ansonsten gesättigt oder ungesättigt und/oder zyklisch, linear oder verzweigt sein. Insbesondere enthält das Kohlenwasserstoffgemisch (KG1) zwischen 10 Gew.-% und 60 Gew. %, mehr bevorzugt zwischen 20 Gew.-% und 50 Gew.-%, MCP und/oder zwischen 1 Gew. % und 30 Gew.-%, mehr bevorzugt zwischen 4 Gew.-% und 20 Gew.-% Benzol. Sofern das Kohlenwasserstoffgemisch (KG1) einen Aromaten, insbesondere Benzol, in den vorstehend angegebenen Mengenbereichen enthält, wird im erfindungsgemäßen Verfahren vorzugsweise vor der Isomerisierung eine Hydrierung durchgeführt, wie sie weiter unten im Zusammenhang mit der Ausführungsform A näher ausgeführt ist.

In einer bevorzugten Ausführungsform der vorliegenden Erfindung enthält das Kohlenwasserstoffgemisch (KG1) Benzol, Methylcyclopentan (MCP) und mindestens eine weitere Verbindung ausgewählt aus Cyclohexan, Cyclopentan, Olefinen oder nicht-cyclischen $C_5$-$C_8$-Alkanen. In dieser Ausführungsform umfassen die weiteren Verbindungen vorzugsweise auch mindestens einen Leichtsieder, ausgewählt aus linearen oder verzweigten $C_5$-Alkanen, Cyclopentan oder linearen oder

verzweigten $C_6$-Alkanen. Der Begriff "Olefin" umfasst neben linearen, einfach ungesättigten Olefinen wie Penten oder Hexen auch cyclische Olefine, insbesondere Cyclohexen, sowie Diene und cyclische Diene. Weiterhin können in der Gruppe der $C_5$-$C_8$-Alkane auch Verbindungen mit einem Normalsiedepunkt > 78 °C, im Folgenden auch "Schwersieder" genannt, enthalten sein. Solche Zusammensetzungen von (KG1) wird vorzugsweise im Zusammenhang mit den weiter unten beschriebenen Ausführungsformen A, B, C und/oder D verwendet, wobei aufgrund der Anwesenheit des Benzols vorzugsweise immer eine Hydrierung durchgeführt wird. Die weiteren Aufreinigungsschritte gemäß der Ausführungsformen A bis D werden nur durchgeführt, sofern bei diesen Zusammensetzungen die optionalen Komponenten Cyclohexan, Schwersieder und/oder Leichtsieder in nennenswertem Umfang auch vorhanden sind.

Besonders bevorzugt enthält das Kohlenwasserstoffgemisch (KG1) Benzol, Methylcyclopentan (MCP) und mindestens einen weiteren Kohlenwasserstoff, ausgewählt aus Cyclohexan, n-Hexan, iso-Hexanen n-Heptan, iso-Heptanen, Methylcyclohexan oder Dimethylcyclopentanen.

In einer weiteren bevorzugten Ausführung von der vorliegenden Erfindung ist im Kohlenwasserstoffgemisch (KG1) zusätzlich Cyclohexan enthalten. Eine solche Zusammensetzung von (KG1) wird vorzugsweise im Zusammenhang mit den weiter unten beschriebenen Ausführungsformen C und D verwendet. Vorzugsweise enthält (KG1)

    i) Benzol,
    ii) MCP,
    iii) DMP,
    iv) Cyclohexan und
    v) gegebenenfalls mindestens eine weitere Verbindung ausgewählt aus Olefinen oder $C_5$-$C_8$-Alkanen.

In der Komponente v) des Kohlenwasserstoffgemisches (KG1) umfasst der Begriff "Olefin" neben linearen, einfach ungesättigten Olefinen wie Penten oder Hexen auch cyclische Olefine, insbesondere Cyclohexen, sowie Diene und cyclische Diene. Weiterhin sind in der Gruppe der $C_5$-$C_8$-Alkane auch Schwersieder mit einem Normalsiedepunkt > 78 °C enthalten. Gegebenenfalls können im Kohlenwasserstoffgemisch (KG1) auch Kohlenwasserstoffe enthalten sein, die mehr als acht Kohlenstoffatome aufweisen und/oder Kohlenwasserstoffe mit einem relativ niedrigen Siedepunkt, beispielsweise solche, die weniger als fünf Kohlenstoffatome aufweisen.

Das Kohlenwasserstoffgemisch (KG1) wird im Rahmen der vorliegenden Erfindung aus einer Vorrichtung zur Aromatenabtrennung gewonnen. Vorrichtungen zur Aromatenabtrennung als solche sind dem Fachmann bekannt, sie können beispielsweise eine, zwei oder noch mehr miteinander verschaltete Destillationsvorrichtungen umfassen. Vorzugsweise wird die Aromatenabtrennung als eine Aromaten-Extraktivdestillation durchgeführt, insbesondere als eine Benzol-Extraktivdestillation. Gegebenenfalls können eine Teilmenge des Kohlenwasserstoffgemisches (KG1) und/oder einzelne darin enthaltene Komponenten aus einer anderen Quelle als der Vorrichtung zur Aromatenabtrennung oder auch aus einem anderen Verfahren als dem Steamcrackverfahren stammen. Beispielsweise können diese Teilmengen und/oder einzelnen Komponenten dem in der Vorrichtung zur Aromatenabtrennung erhaltenen Kohlenwasserstoffgemisch (KG1) anschließend zugegeben werden.

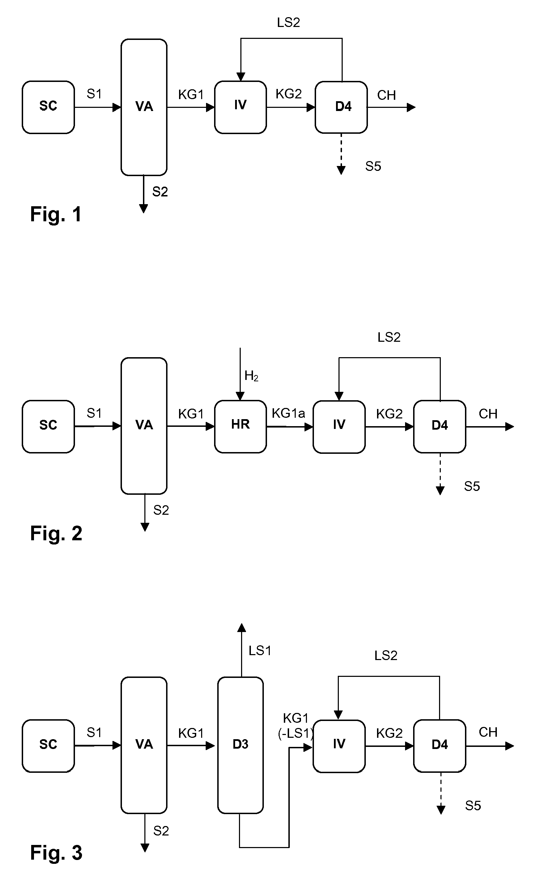

Die Vorrichtung zur Aromatenabtrennung wiederum ist einem Steamcrackverfahren nachgeschaltet. Ein aus dem Steamcrackverfahren stammender Strom (S1) wird in die Vorrichtung zur Aromatenabtrennung eingespeist. In der Vorrichtung zur Aromatenabtrennung wird der Strom (S1) in einen aromatenreichen Strom (S2) sowie in das Kohlenwasserstoffgemisch (KG1) aufgetrennt. Dies ist im nachfolgenden Text im Zusammenhang mit der Figur 1 nochmals verdeutlicht.

Die Durchführung eines Steamcrackverfahren als solchem ist dem Fachmann bekannt. Das Steamcrackverfahren ist in seinen Grundzügen bereits in der Einleitung der vorliegenden Erfindung beschrieben worden, worauf Bezug genommen wird. Vorzugsweise handelt es sich im Rahmen der vorliegenden Erfindung bei dem Steamcrackverfahren um einen Naphtha-Cracker (Naphtha-Steamcrackverfahren). Der Strom (S1) stammt also vorzugsweise aus einem Naphtha-Cracker und/oder der Strom (S1) umfasst Pyrolysebenzin oder einen aus dem Pyrolysebenzin abgetrennten Teilstrom.

Der Strom (S1) wird auch als Zustrom (S1) zur Vorrichtung zur Aromatenabtrennung bezeichnet. Der Strom (S1) umfasst das Kohlenwasserstoffgemisch (KG1) und zusätzlich einen Anteil an Aromaten. Diese zusätzlichen Aromaten werden in der Vorrichtung zur Aromatenabtrennung also vom Kohlenwasserstoffgemisch (KG1) abgetrennt. Dies bedeutet weiterhin, dass das Kohlenwasserstoffgemisch (KG1) eine geringere Konzentration an Aromaten, insbesondere Benzol, aufweist als der Zustrom (S1) zur Vorrichtung zur Aromatenabtrennung, beispielsweise kann das Kohlenwasserstoffgemisch (KG1) eine um mindestens 50% geringere Konzentration an Aromaten aufweisen als der Zustrom (S1) zur Vorrichtung zur Aromatenabtrennung.

Wie in der Einleitung im Zusammenhang mit dem Pyrolysebenzin bereits ausgeführt, kann im Rahmen der vorliegenden Erfindung der Aromatentrennung auch eine Auftrennung in jeweils an Benzol, Toluol und Xylole angereicherte Fraktionen, gegebenenfalls ergänzt durch weitere Verfahrensschritte, vorgeschaltet sein. In diesem Fall ist die an Benzol angereicherte Fraktion als Strom (S1) zu verstehen.

Die an Benzol angereicherte Fraktion wird dann bevorzugt mittels Extraktivdestillation, beispielsweise unter Verwendung von N-Formyl-Morpholin als Hilfsstoff, in einen Benzol in hoher Reinheit enthaltenden Strom und einen an Benzol abgereicherten Strom, der auch als C6-Nichtaromatenstrom (C6-NA) bezeichnet wird, aufgetrennt. In diesem Fall ist das Kohlenwasserstoffgemisch (KG1) gemäß der vorliegenden Erfindung mit dem C6-Nichtaromatenstrom (C6-NA) gleichzusetzen.

C6-NA kann enthalten:

- lineare offenkettige und/oder verzweigte sowie zyklische (naphthenische) C5-Kohlenwasserstoffe wie z.B. n-Pentan, Isopentane, Cyclopentan,
- lineare offenkettige und/oder verzweigte sowie zyklische (naphthenische) C6-Kohlenwasserstoffe wie z.B. n-Hexan, Isohexane, Methylcyclopentan (MCP), Cyclohexan.
- lineare offenkettige und/oder verzweigte sowie zyklische (naphthenische) C7-Kohlenwasserstoffe wie z.B. n-Heptan, Isoheptane wie z.B. Dimethylpentane (DMP), Methylcyclohexan (MCP), Dimethylcyclopentane.
- Olefine und/oder Aromaten, deren Struktur von einer oder mehreren der vorgenannten Kohlenwasserstoffe mittels Eliminierung von Wasserstoff abgeleitet ist, wie z. B. Benzol oder Cyclohexen.

Wie in der Einleitung weiterhin bereits ausgeführt, kann die Aromatenabtrennung in Abhängigkeit von der konkreten Zusammensetzung des aus dem Steamcrackverfahren stammenden aromatenreichen Stromes in weiten Grenzen variieren. Beispielsweise ist es möglich, dass bereits bei der Aromatenabtrennung sämtliche Aromaten inklusive Benzol von den sonstigen Kohlenwasserstoffen abgetrennt werden. In der Regel wird aber die Aromatenabtrennung so ausgeführt und betrieben, dass zwar die Hauptmenge des im Strom (S1) enthaltenen Benzol zusammen mit anderen Aromaten über den Strom (S2) aus dem Verfahren abgezogen wird, aber eine Teilmenge des Benzols im Kohlenwasserstoffgemisch (KG1) verbleibt. Sinngemäß gilt auch für andere Aromaten, die im Kohlenwasserstoffgemisch (KG1) enthalten sein können.

Die Isomerisierung von MCP zu Cyclohexan gemäß Schritt a) erfolgt in Gegenwart eines Katalysators. Als Katalysatoren eignen sich prinzipiell alle dem Fachmann hierfür bekannten Katalysatoren, beispielsweise Friedel-Crafts-Katalysatoren gemäß US-A 2,846,485 wie Aluminiumchlorid, das zusätzlich HCl enthalten kann, oder Metallhalogeniden gemäß US-A 3,311,667 wie Aluminiumchlorid, Zirkoniumchlorid oder Bortrifluorid. Weiterhin eignen sich als Katalysatoren auch die in EP-A 1 995 297 verwendeten Zeolithe oder ionische Flüssigkeiten wie sie beispielsweise in WO 2011/069929 verwendet werden.

Im Rahmen der vorliegenden Erfindung erfolgt die Isomerisierung vorzugsweise in Gegenwart einer sauren ionischen Flüssigkeit mit der Zusammensetzung $K1Al_nX_{(3n+1)}$, wobei K1 ein einwertiges Kation, X gleich Halogen und 1 < n < 2,5 ist. Beispielsweise können Gemische aus zwei oder mehr sauren ionischen Flüssigkeiten eingesetzt werden, vorzugsweise wird eine saure ionische Flüssigkeit eingesetzt.

K1 ist vorzugsweise ein unsubstituiertes oder zumindest teilweise alkyliertes Ammoniumion oder ein heterocyclisches (einwertiges) Kation, insbesondere ein Pyridiniumion, ein Imidazoliumion, ein Pyridaziniumion, ein Pyrazoliumion, ein Imidazoliniumion, ein Thiazoliumion, ein Triazoliumion, ein Pyrrolidiniumion, ein Imidazolidiniumion oder ein Phosphoniumion. X ist vorzugsweise Chlor oder Brom.

Mehr bevorzugt enthält die saure ionische Flüssigkeit als Kation ein zumindest teilweise alkyliertes Ammoniumion oder ein heterocyclisches Kation und/oder als Anion ein Chloroaluminat mit der Zusammensetzung $Al_nCl_{(3n+1)}$ mit 1 < n < 2,5. Vorzugsweise enthält das zumindest teilweise alkylierte Ammoniumion einen, zwei oder drei Alkylreste mit (jeweils) ein bis zehn Kohlenstoffatomen. Sofern zwei oder drei Alkylsubstituenten mit den entsprechenden Ammoniumionen vorhanden sind, kann die jeweilige Kettenlänge unabhängig voneinander gewählt werden, vorzugsweise weisen alle Alkylsubstituenten die gleiche Kettenlänge auf. Besonders bevorzugt sind trialkylierte Ammoniumionen mit einer Kettenlänge von ein bis drei Kohlenstoffatomen. Das heterocyclische Kation ist vorzugsweise ein Imidazoliumion oder ein Pyridiniumion.

Besonders bevorzugt enthält die saure ionische Flüssigkeit als Kation ein zumindest teilweise alkyliertes Ammoniumion und als Anion ein Chloroaluminat mit der Zusammensetzung $Al_nCl_{(3n+1)}$ mit 1 < n < 2,5. Beispiele für solche besonders bevorzugten sauren ionischen Flüssigkeiten sind Trimethylammoniumchloroaluminat und Triethylammoniumchloroaluminat.

Weiterhin kann bei der Isomerisierung zusätzlich zur sauren ionischen Flüssigkeit auch ein Wasserstoffhalogenid (HX) als Cokatalysator verwendet werden. Als Wasserstoffhalogenid (HX) können prinzipiell alle denkbaren Wasserstoffhalogenide eingesetzt werden, beispielsweise Fluorwasserstoff (HF), Chlorwasserstoff (HCl), Bromwasserstoff (HBr) oder Iodwasserstoff (HI). Gegebenenfalls können die Wasserstoffhalogenide auch als Gemisch eingesetzt werden, vorzugsweise wird im Rahmen der vorliegenden Erfindung jedoch nur ein Wasserstoffhalogenid eingesetzt. Vorzugsweise wird das Wasserstoffhalogenid verwendet, dessen Halogenidteil auch in der vorstehend beschriebenen sauren ionischen Flüssigkeit (zumindest teilweise) im entsprechenden Anion enthalten ist. Vorzugsweise ist das Wasserstoffhalogenid (HX) Chlorwasserstoff (HCl) oder Bromwasserstoff (HBr). Besonders bevorzugt ist das Wasserstoffhalogenid (HX) Chlorwasserstoff (HCl).

Als Vorrichtung (IV) zur Durchführung der Isomerisierung können prinzipiell alle dem Fachmann für einen solchen Zweck bekannten Vorrichtungen verwendet werden. Vorzugsweise ist die Vorrichtung (IV) ein Rührkessel oder eine Rührkesselkaskade. Rührkesselkaskade bedeutet, dass zwei oder mehr, beispielsweise drei oder vier, Rührkessel hintereinander (in Reihe) geschaltet sind.

Die Isomerisierung wird bevorzugt bei einer Temperatur zwischen 0 °C und 100 °C, besonders bevorzugt bei einer Temperatur zwischen 30 °C und 60 °C durchgeführt. Weiterhin ist es bevorzugt, dass der Druck bei der Isomerisierung zwischen 1 und 20 bar abs. (absolut), bevorzugt zwischen 2 und 10 bar abs., beträgt.

Die Durchführung der Isomerisierung gemäß Schritt a), vorzugsweise einer Isomerisierung von MCP in Gegenwart einer sauren ionischen Flüssigkeit als Katalysator sowie gegebenenfalls einem Wasserstoffhalogenid als Cokatalysator ist den Fachmann bekannt. Vorzugsweise bilden die Kohlenwasserstoffe (also MCP und gegebenenfalls sonstige in (KG1) enthaltenen Kohlenwasserstoffe) und die ionische Flüssigkeit bei der Isomerisierung jeweils eine separate Phase aus, wobei Teilmengen der ionischen Flüssigkeit in der Kohlenwasserstoffphase und Teilmengen der Kohlenwasserstoffe in der ionischen Flüssigkeitsphase enthalten sein können. Sofern vorhanden, wird das Wasserstoffhalogenid, insbesondere Chlorwasserstoff, vorzugsweise gasförmig in die Vorrichtung (IV) zur Durchführung der Isomerisierung eingeleitet. Das Wasserstoffhalogenid kann, zumindest in Teilmengen, in den beiden vorgenannten Flüssigkeitsphasen sowie in einer, vorzugsweise zusätzlich vorhandenen, gasförmigen Phase enthalten sein.

Vorzugsweise wird die Isomerisierung in der Vorrichtung (IV) so durchgeführt, dass in einem Rührkessel oder einer Rührkesselkaskade zwei flüssige Phasen und eine Gasphase vorliegen. Die erste flüssige Phase enthält zu mindestens 90 Gew.-% die saure ionische Flüssigkeit und die zweite flüssige Phase enthält zu mindestens 90 Gew.-% die Kohlenwasserstoffe. Die Gasphase enthält zu mindestens 90 Gew.-% mindestens ein Wasserstoffhalogenid, vorzugsweise Chlorwasserstoff. Gegebenenfalls kann auch noch eine feste Phase vorhanden sein, die Komponenten in fester Form enthält, aus denen die ionische Flüssigkeit gebildet wird, wie beispielsweise AlCl$_3$. Dabei werden Druck und Zusammensetzung der Gasphase so eingestellt, dass der Partialdruck des gasförmigen Wasserstoffhalogenid, insbesondere von HCl-Gas, in der Gasphase zwischen 0,5 und 20 bar abs. (absolut), bevorzugt zwischen 1 und 10 bar abs. beträgt.

Weiterhin ist es im Rahmen der vorliegenden Erfindung bevorzugt, dass die Isomerisierung in einer Dispersion (D1) durchgeführt wird, wobei in der Dispersion (D1) die Phase (B) in der Phase (A) dispergiert ist, das Volumenverhältnis der Phase (A) zu Phase (B) im Bereich von 2,5 bis 4 zu 1 [Vol/Vol] liegt, die Phase (A) zu > 50 Gew.-% mindestens eine saure ionische Flüssigkeit enthält und die Phase (B) zu > 50 Gew.-% mindestens einen nichtaromatischen Kohlenwasserstoff enthält. Darüber hinaus ist es bevorzugt, dass die Dispersion (D1) zusätzlich HCl enthält und/oder gasförmiges HCl in die Dispersion (D1) eingeleitet wird.

Wie vorstehend bereits ausgeführt, wird in der Isomerisierung in Gegenwart eines Katalysators, vorzugsweise einer sauren ionischen Flüssigkeit und gegebenenfalls eines Wasserstoffhalogenids (HX), MCP zumindest teilweise zu Cyclohexan isomerisiert. Gegebenenfalls können noch weitere in (KG1) enthaltene Kohlenwasserstoffe außer MCP isomerisiert werden. Die bei der Isomerisierung erhaltenen Kohlenwasserstoffe sind in dem Kohlenwasserstoffgemisch (KG2) enthalten. Das Gemisch (KG2) unterscheidet sich also hinsichtlich der Zusammensetzung und/oder Menge der darin enthaltenen Kohlenwasserstoffe von dem entsprechenden Kohlenwasserstoffgemisch (KG1), das vor der Isomerisierung vorliegt. Das Kohlenwasserstoffgemisch (KG1) wurde bereits vorstehend definiert.

Da bei solchen Isomerisierungsprozessen die durchzuführende Isomerisierung meist nicht zu 100 % (also vollständig) abläuft, ist im Produkt in aller Regel auch der Kohlenwasserstoff, mit dem die Isomerisierung durchgeführt wurde (in geringerer Menge als vor der Isomerisierung), noch enthalten. Da im vorliegenden Fall MCP zu Cyclohexan isomerisiert wird, ist im Isomerisierungsprodukt in der Regel ein Gemisch aus Cyclohexan und (in geringerer Menge als vor der Isomerisierung) MCP enthalten.

Alle Komponenten des Kohlenwasserstoffgemischs (KG1), die in Schritt a) nicht isomerisiert werden, sind jedoch ebenfalls im Kohlenwasserstoffgemisch (KG2) enthalten. Dabei ist aber zu berücksichtigen, dass vor der Isomerisierung gemäß Schritt a) zusätzliche Zwischenschritte mit dem aus der Vorrichtung zur Aromatenabtrennung stammenden Kohlenwasserstoffgemisch (KG1) durchgeführt werden können. Solche Zwischenschritte sind im nachfolgenden Text beispielsweise als Ausführungsformen A bis C dargestellt. Sofern einer oder mehrerer dieser Zwischenschritte durchgeführt wird, erfolgt die Isomerisierung (wie weiter unten dargelegt) nicht unter Verwendung von (KG1), sondern beispielsweise mit den Kohlenwasserstoffgemischen (KG1a) oder (KG1b). Sofern dies der Fall ist, muss stattdessen die Zusammensetzung von (KG2) in Relation zu den Kohlenwasserstoffgemischen (KG1 a) oder (KG1 b) gesetzt werden.

In Schritt b) des erfindungsgemäßen Verfahrens erfolgt die Isolierung von Cyclohexan aus dem Kohlenwasserstoffgemisch (KG2).

Die Isolierung des Cyclohexans kann nach dem Fachmann bekannten Methoden beispielsweise unter Verwendung von einer oder mehreren Destillationskolonnen erfolgen, in die der Austrag der Vorrichtung eingeleitet wird, in der die Isomerisierung gemäß Schritt a) durchgeführt wurde. Vorzugsweise erfolgt die Isolierung des Cyclohexans gemäß Schritt b) unter Verwendung von mindestens einer Destillationskolonne, insbesondere unter Verwendung von mindestens einer Rektifikationskolonne. Die nachfolgend beschriebenen Kolonnen/Vorrichtungen (D4) bis (D6) sind also jeweils vorzugsweise als Rektifikationskolonnen ausgestaltet. In der Regel wird im erfindungsgemäßen Verfahren im Anschluss an die Isomerisierung Cyclohexan in einer Reinheit von mindestens 98 Gew.-%, vorzugsweise von mindestens 99,5 Gew.-%, besonders bevorzugt von mindestens 99,9 Gew.-% isoliert.

Vorzugsweise wird Schritt b) des erfindungsgemäßen Verfahrens so durchgeführt, dass das Kohlenwasserstoffgemisch (KG2) enthaltend Cyclohexan, MCP, gegebenenfalls nicht-cyclische $C_5$-$C_6$-Alkane und gegebenenfalls schwerer als Cyclohexan siedende Komponenten in eine Destillationskolonne (D4) eingespeist wird, wobei aus (D4) an einer Entnahmestelle oberhalb des Zulaufs, bevorzugt über Kopf, der Großteil des im Zulauf zu (D4) enthaltenen MCP und gegebenenfalls an nichtcyclischen $C_5$-$C_6$-Alkanen abgetrennt wird. Sofern in (KG2) nicht-cyclische $C_5$-$C_6$-Alkane vorhanden sind, handelt es sich dabei vorzugsweise um $C_6$-Alkane, mehr bevorzugt um n-Hexan und iso-Hexane. Dieser den Großteil an MCP (und gegebenenfalls an nicht-cyclischen $C_5$-$C_6$-Alkanen) enthaltende Strom wird nachfolgend auch als Strom (LS2) bezeichnet.

Der Strom (LS2 ) ist weiterhin dadurch charakterisiert, dass er (bezogen auf (KG2)) an MCP angereichert und an Cyclohexan abgereichert ist, wobei dieser Strom (LS2) bevorzugt weniger als 20 Gew.-%, bevorzugt weniger als 10 Gew.-%, besonders bevorzugt weniger als 7 Gew.-% Cyclohexan enthält. Weiterhin ist es bevorzugt, dass der Strom (LS2) in oder vor die Vorrichtung zur Isomerisierung zurückgeführt wird.

Der Begriff "Rückführung vor die Vorrichtung zur Isomerisierung" kann erfindungsgemäß bedeuten, dass der Strom (LS2) ganz oder teilweise auch in oder vor Verfahrensschritte, bzw. die entsprechenden Vorrichtungen zur Durchführung dieser Verfahrensschritte, rückgeführt werden kann, die wiederum selber der Isomerisierung vorgeschaltet sind. Bei solchen Verfahrensschritten handelt es sich beispielsweise um die Leichtsiederabtrennung gemäß der nachfolgend beschriebenen Ausführungsform B. Der Strom (LS2) kann also ganz oder teilweise in oder vor die Vorrichtung zur Durchführung der Leichtsiederabtrennung rückgeführt werden.

Das Cyclohexan kann aus der Destillationskolonne (D4), vorzugsweise sofern keine schwerer als Cyclohexan siedenden Komponenten in die jeweilige Spezifikation beeinträchtigender Konzentration vorliegen, in einer Reinheit von mindestens 98 Gew.-%, vorzugsweise von mindestens 99,5 Gew. %, besonders bevorzugt von mindestens 99,9 Gew.-%, über den Sumpf von (D4) oder einen unterhalb des Zulaufs gelegenen Seitenabzug von (D4), vorzugsweise dampfförmigen Seitenabzug von (D4), abgezogen werden (Option b0)).

Alternativ kann auch die Option b1) verwirklicht werden, wobei der über den Sumpf von (D4) abgezogene an Cyclohexan angereicherte Strom in eine Destillationskolonne (D5) eingeleitet wird, wobei über den Sumpf von (D5) ein Strom (S5), enthaltend schwerer als Cyclohexan siedende Komponenten, abgetrennt wird und über eine Abnahmestelle oberhalb des Zulaufs zu (D5), bevorzugt über Kopf, Cyclohexan mit einer Reinheit von mindestens 98 Gew.-%, vorzugsweise von mindestens 99,5 Gew. %, besonders bevorzugt von mindestens 99,9 Gew.-%, abgezogen wird.

Alternativ kann auch die Option b2) verwirklicht werden, wobei ein an Cyclohexan angereicherter Strom, der bevorzugt dampfförmig ist, über den Seitenabzug aus der Destillationskolonne (D4) abgetrennt wird, wobei sich der Seitenabzug vorzugsweise im Abtriebsteil von (D4) befindet und/oder der an Cyclohexan angereicherte Strom aus dem Seitenabzug von (D4) in eine bevorzugt als Destillationskolonne ausgeführte Vorrichtung (D6) zur weiteren Aufreinigung geleitet wird und dort über eine Abnahmestelle oberhalb des Zulaufs zu (D6), bevorzugt über Kopf, Cyclohexan mit einer Reinheit von mindestens 98 Gew.-%, vorzugsweise von mindestens 99,5 Gew. %, besonders bevorzugt von mindestens 99,9 Gew.-%, gewonnen wird.

Dabei ist es in der Option b2) weiterhin bevorzugt, dass der Zulauf des bevorzugt dampfförmigen Stromes aus (D4) nach (D6) unterhalb des untersten Bodens, der untersten Packung oder der untersten Füllkörperschüttung von (D6) erfolgt und (D6) mit einem Kopfkondensator und teilweisem Rücklauf des aus diesem abgezogenen Kondensats, nicht aber mit einem eigenen Sumpfverdampfer betrieben wird und dass die unten in (D6) anfallende Flüssigkeit ungefähr in Höhe des Seitenabzugs in die Destillationskolonne (D4) zurückgeleitet wird. In dieser Ausführungsform wird ein schwerer als Cyclohexan siedende Komponenten enthaltender Strom (S5) über den Sumpf von (D4) abgezogen.

Alternativ kann auch die Option b3) verwirklicht werden, wobei die Destillationskolonne (D4) als Trennwandkolonne ausgeführt ist, die Trennwand sich teilweise unterhalb der Zulaufstelle befindet, eine Abzugsstelle im Bereich der Trennwand liegt und über diese Abzugsstelle ein bevorzugt flüssiger Cyclohexan-Strom mit einer Reinheit von mindestens 98 Gew.-%, vorzugsweise von mindestens 99,5 Gew. %, besonders bevorzugt von mindestens 99,9 Gew.-% entnommen wird. In dieser Ausführungsform wird ebenfalls ein schwerer als Cyclohexan siedende Komponenten enthaltender Strom (S5) über den Sumpf von (D4) abgezogen.

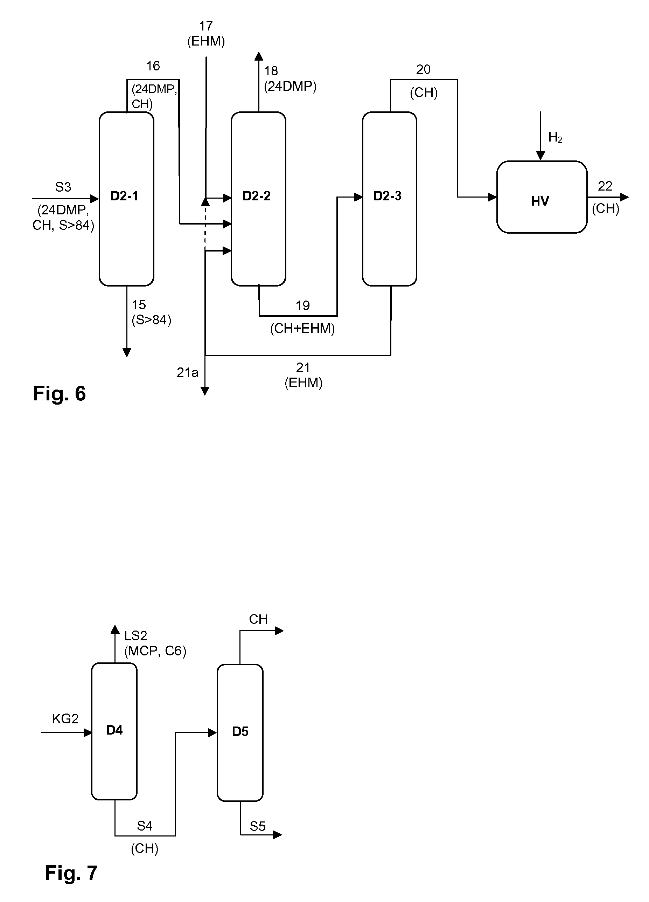

In Figur 7 wird Schritt b) des erfindungsgemäßen Verfahrens gemäß der vorstehend beschriebenen Option b1) nochmals verdeutlicht. CH bedeutet Cyclohexan, C6 bedeutet nicht-cyclische $C_5$-$C_6$-Alkane, insbesondere iso-Hexane und die in Klammer gesetzten Ausdrücke geben die für das Verfahren relevantesten und/oder die Hauptkomponenten des jeweiligen Stroms an. In der Ausführungsform gemäß Figur 7 wird ein Kohlenwasserstoffgemisch (KG2) eingesetzt, in dem Cyclohexan, MCP, nichtcyclische $C_5$-$C_6$-Alkane, insbesondere n-Hexan, und Schwersieder mit einem Normalsiedepunkt > 84 °C enthalten sind. Aus dem Sumpf von D4 wird ein an Cyclohexan angereicherter Strom (S4) in die Destillationskolonne (D5) eingeleitet, aus der über Kopf spezifikationsgerechtes Cyclohexan isoliert wird. Der Sumpfstrom (S5) umfasst schwerer als Cyclohexan siedende Komponenten.

Gegebenenfalls werden im Rahmen der vorliegenden Erfindung im Anschluss an die Isomerisierung gemäß Schritt a) und vor einer destillativen Abtrennung/Isolierung des Cyclohexans gemäß Schritt b) zusätzliche Aufreinigungsschritte mit dem Austrag der Isomerisierung durchgeführt. Bei diesen Aufreinigungsschritten kann es sich beispielsweise um eine neutrale und/oder alkalische Wäsche handeln, die einstufig oder mehrstufig durchgeführt werden kann. Zusätzlich oder alternativ zur Wäsche können auch spezielle Vorrichtungen, beispielsweise Destillations- oder Rektifiziervorrichtungen, verwendet werden, um beispielsweise vorhandenes Wasserstoffhalogenid von den Kohlenwasserstoffen abzutrennen. Solche Vorrichtungen umfassen auch Vorrichtungen zur einstufigen Verdampfung, insbesondere zur Flash-Verdampfung. Zusätzlich oder alternativ können im Falle der Verwendung von saurer ionischer Flüssigkeit auch Phasentrenneinheiten, vorzugsweise Phasenscheider, den vorgenannten speziellen Vorrichtungen vorgeschaltet werden, insbesondere um die saure ionische Flüssigkeit von den Kohlenwasserstoffen abzutrennen.

In einer besonders bevorzugt Ausführungsform wird die Isomerisierung in Gegenwart von saurer ionischer Flüssigkeit durchgeführt und der Austrag aus der Isomerisierung in eine Phasentrenneinrichtung wie z. B. einen Phasenscheider geführt, wo eine Auftrennung in eine zu mindestens 90 Gew.% aus saurer ionischer Flüssigkeit bestehende Phase und eine zu mindestens 90 Gew.% aus Kohlenwasserstoffen bestehende Phase durchgeführt wird. Die zu mindestens 90 Gew.% aus saurer ionischer Flüssigkeit bestehende Phase wird zumindest teilweise in die Isomerisierung zurückgeführt und der zu mindestens 90 Gew.% aus Kohlenwasserstoffen bestehende Phase wird, nachdem ihr gegebenenfalls in einer Destillations- oder Rektifiziervorrichtung leichtflüchtige Bestandteile wie z. B. HCl entzogen worden sind, in eine neutrale und/oder alkalische Wäsche geführt, wo Reste der ionischen Flüssigkeit oder Bestandteile derselben wie z. B. HCl oder $AlCl_3$ entfernt werden.

In Figur 1 wird das erfindungsgemäße Verfahren in seiner Grundform unter Berücksichtigung der Schritte a) und b) nochmals verdeutlicht. CH bedeutet Cyclohexan, SC bedeutet Steamcrackverfahren bzw. Vorrichtungen, in denen ein solches Verfahren durchgeführt wird, VA bedeutet Vorrichtung zur Aromatenabtrennung und IV bedeutet Isomerisierungsvorrichtung. Das Kohlenwasserstoffgemisch (KG1) enthält mindestens MCP. Sofern (KG1) zusätzlich mindestens einen Aromaten, insbesondere Benzol, mindestens einen Schwersieder, insbesondere DMP, und/oder mindestens einen Leichtsieder, insbesondere Isohexane enthält, wird anstelle der vorliegend in Figur 1 beschriebenen Grundform des erfindungsgemäßen Verfahrens vorzugsweise eine der in den nachfolgenden Figuren 2 bis 6 verdeutlichten bevorzugten Ausführungsformen des erfindungsgemäßen Verfahrens durchgeführt.

Im Verfahren gemäß Figur 1 wird zunächst aus einem vorgeschalteten Steamcrackverfahren ein Strom (S1) gewonnen, der Aromaten, bevorzugt Benzol, und darüber hinaus die Komponenten des Kohlenwasserstoffgemischs (KG1) enthält. Dieser Strom (S1) wird in eine Vorrichtung zur Aromatentrennung (VA) eingespeist. (VA) wird vorzugsweise als eine Aromaten-Extraktivdestillation, insbesondere als Benzol-Extraktivdestillation, betrieben. Aus (VA) werden ein aromatenreicher, bevorzugt ein benzolreicher, Strom (S2) sowie das Kohlenwasserstoffgemisch (KG1) abgetrennt.

In Schritt a) wird das Kohlenwasserstoffgemisch (KG1) einer Isomerisierung unter Erhalt des Kohlenwasserstoffgemischs (KG2) unterzogen, wobei MCP zumindest teilweise in Cyclohexan umgewandelt wird. Die Isomerisierung des Kohlenwasserstoffgemischs (KG1) erfolgt in einer hierfür geeigneten Isomerisierungsvorrichtung (IV). Vorzugsweise wird als Katalysator bei der Isomerisierung eine saure ionische Flüssigkeit eingesetzt, wobei, besonders bevorzugt, die Isomerisierung in einem Rührkessel oder einer Rührkesselkaskade durchgeführt wird.

Aus dem Isomerisierungsprodukt wird anschließend gemäß Schritt b) Cyclohexan isoliert, beispielsweise unter Verwendung von einer oder mehreren Destillationskolonnen, in die der Austrag der Isomerisierungsvorrichtung (IV) eingeleitet wird; dort wird Cyclohexan von nicht umgesetztem MCP und gegebenenfalls weiteren Komponenten abgetrennt, wobei der an MCP angereicherte und an Cyclohexan abgereicherte Teilstrom bevorzugt vor oder in die Isomerisierungsvorrichtung (IV) zurückgeführt wird. Schritt b) ist in Figur 1 vereinfacht durch die Destillationsvorrichtung (D4) dargestellt. Vorzugsweise wird Schritt b) so durchgeführt, wie vorstehend im Zusammenhang mit Figur 7 dargestellt. Demzufolge ist in Figur 1 die optionale Abtrennung von schwerer als Cyclohexan siedenden Komponenten über den Strom (S5) als mögliche Variante durch den gestrichelten Pfeil angedeutet.

Sofern (KG1) zusätzlich mindestens einen Aromaten, insbesondere Benzol enthält, kommt eine bevorzugte Ausführungsform des erfindungsgemäßen Verfahrens zur Anwendung (nachfolgend auch als "Ausführungsform A" bezeichnet) in der das Verfahren zusätzlich den Schritt c) umfasst, der vor Schritt a) durchgeführt wird, mit

c) Hydrierung des Kohlenwasserstoffgemisches (KG1), enthaltend mindestens MCP und mindestens einen Aromaten, unter Erhalt eines Kohlenwasserstoffgemisches (KG1a), das eine gegenüber (KG1) reduzierte Menge an mindestens einem Aromaten aufweist, wobei in den nachfolgenden Schritten (KG1 a) statt (KG1) verwendet wird.

In anderen Worten ausgedrückt bedeutet dies, dass in Schritt c) die in dieser Ausführungsform im Kohlenwasserstoffgemisch (KG1) enthaltenen Aromaten unter Erhalt der entsprechenden nichtaromatischen Kohlenwasserstoffe, vorzugsweise der sich unter Erhalt aller Kohlenstoff-Kohlenstoff-Bindungen ergebenden vollständig gesättigten Kohlenwasserstoffe, hydriert werden. Sofern im Kohlenwasserstoffgemisch (KG1) sonstige ungesättige Verbindungen enthalten sind, beispielsweise Olefine wie Cyclohexen, werden diese in Schritt c) der vorliegenden Erfindung ebenfalls hydriert. Vorzugsweise enthält das Kohlenwasserstoffgemisch (KG1) als Aromat Benzol und/oder das Kohlenwasserstoffgemisch (KG1a) weist eine gegenüber (KG1) erhöhte Menge an Cyclohexan auf.

Die Hydrierung des Kohlenwasserstoffgemischs (KG1) gemäß Ausführungsform A erfolgt im Rahmen der vorliegenden Erfindung in einer hierfür geeigneten Vorrichtung (V), die vorzugsweise mindestens einen Hydrierreaktor (HR) umfasst. In der Vorrichtung (V) wird Benzol zu Cyclohexan hydriert, wobei die Hydrierung vorzugsweise unter Verwendung von elementarem Wasserstoff erfolgt. Weiterhin ist es bevorzugt, dass die Hydrierung in flüssiger Phase erfolgt.

Die Hydrierung von mindestens einem Aromaten gemäß Schritt c), vorzugsweise von Benzol zu Cyclohexan, wird in der Regel in Gegenwart eines geeigneten Katalysators durchgeführt. Als Katalysatoren eignen sich prinzipiell alle dem Fachmann hierfür bekannten Katalysatoren, beispielsweise ein Metallkatalysator auf Kieselgur gemäß US-A 3,311,667 oder metallhaltige Katalysatoren gemäß EP A 1 995 297, wobei dort als Metall die Elemente der Platingruppe, Zinn oder Kobalt und Molybdän bevorzugt verwendet werden.

Vorzugsweise wird die Hydrierung in Gegenwart eines Katalysators durchgeführt, der als Aktivmetall (auch als Metallkomponente beziehungsweise Aktivkomponente bezeichnet) mindestens ein Element der 8. bis 10. Gruppe des Periodensystems der Elemente (PSE), beispielsweise Eisen, Cobalt, Nickel oder Ruthenium (entspricht der Nebengruppe VIIIB der CAS-Version des PSE) enthält, insbesondere Nickel oder Ruthenium. Weiterhin ist es bevorzugt, dass das Aktivmetall auf einem Trägermaterial (Träger) aufgebracht ist. Als Träger eignen sich prinzipiell alle dem Fachmann bekannten Träger, beispielsweise $SiO_2$-haltige, zirconiumoxidhaltige oder aluminiumoxidhaltige Träger. Besonders bevorzugt wird ein Katalysator eingesetzt, der Nickel als Aktivmetall auf einem aluminiumoxidhaltigen Träger enthält.

Die Hydrierung als solche wird in der dem Fachmann an sich bekannten Art ausgeführt und betrieben, bevorzugt ist eine Kombination aus einem im gegebenenfalls gekühlten Kreislauf betriebenen Hauptreaktor (Rückführung eines Teils des aus dem Reaktor ausströmenden Gemischs in das dem Reaktor zuströmende Gemisch, wobei gegebenenfalls die Kühlung vor oder nach der genannten Zuführung platziert ist) und einem nachfolgenden im geraden Durchgang, das heißt ohne Rückführung betriebenen Nachreaktor. In diesem Fall umfasst die Vorrichtung (V) also zwei Hydrierreaktoren (HR).

Die Hydrierreaktoren (HR) sind bevorzugt als Festbettreaktoren ohne interne Kühlung ausgelegt. In diesem Fall wird die Hydrierung bevorzugt so betrieben, dass die Temperaturdifferenz zwischen eintretendem und austretendem Gemisch kontinuierlich überwacht und bei Absinken dieses Werts unter einen bestimmten Sollwert die Eintrittstemperatur angehoben wird. Weiterhin ist bevorzugt, dass die Hydrierreaktoren in Rieselfahrweise betrieben werden.

Weiterhin bevorzugt wird der Hydrierung ein Apparat nachgeschaltet, in dem auf einen Druck unterhalb des im Nachreaktor eingestellten Drucks entspannt wird. Dabei fällt ein Gasstrom an, der zuvor im Kohlenwasserstoffgemisch eingelösten Wasserstoff enthält und jedenfalls verdichtet und in mindestens einen der Hydrierreaktoren (HR) zurückgeführt wird.

Die Hydrierung wird bevorzugt bei einer Temperatur zwischen 50 und 200 °C, besonders bevorzugt zwischen 100 und 180 °C und/oder einem Druck zwischen 10 und 300 bar abs., besonders bevorzugt zwischen 30 und 200 bar abs. durchgeführt. Weiterhin ist es im erfindungsgemäßen Verfahrens bei der Hydrierung bevorzugt, dass der Gesamtumsatz der Aromaten, insbesondere des Benzols (und gegebenenfalls sonstiger im Kohlenwasserstoffgemisch (KG1) enthaltener ungesättigter Verbindungen) mindestens 90 %, besonders bevorzugt 99 % beträgt und/oder der Restgehalt der Aromaten, insbesondere des Benzols (und gegebenenfalls sonstiger im Kohlenwasserstoffgemisch (KG1) enthaltenen ungesättigten Verbindungen) im Kohlenwasserstoffgemisch (KG1a) 1 Gew.-%, bevorzugt höchstens 0,1 Gew.-%, besonders bevorzugt höchstens 0,01 Gew.-% beträgt.

Infolge der Hydrierung wird im erfindungsgemäßen Schritt c) das Kohlenwasserstoffgemisch (KG1a) erhalten, das sich in seiner Zusammensetzung vom Kohlenwasserstoffgemisch (KG1) vordergründig hinsichtlich der hydrierten Verbindungen unterscheidet. Das Kohlenwasserstoffgemisch (KG1a) enthält also mindestens einen durch Hydrierung eines Aromaten gebildeten Kohlenwasserstoff und mindestens MCP, das bereits in (KG1) enthalten gewesen ist. Weiterhin enthält das Kohlenwasserstoffgemisch (KG1a) alle sonstigen Komponenten gemäß dem Kohlenwasserstoffgemisch (KG1), die bei der Hydrierung chemisch nicht geändert werden, sowie gegebenenfalls durch Hydrierung von Olefinen oder Dienen gebildete Kohlenwasserstoffe. Sofern im Kohlenwasserstoffgemisch (KG1) als Aromat Benzol enthalten ist, enthält das Kohlenwasserstoffgemisch (KG1a) entsprechend Cyclohexan. Sofern vor der Hydrierung gemäß Ausführungsform A eine Schwersieder-Abtrennung gemäß Ausführungsform C (siehe die entsprechenden Textpassagen) durchgeführt wird, wird zur Hydrierung anstelle des Kohlenwasserstoffgemisches (KG1) das entsprechende Kohlenwasserstoffgemisch (KG1 b) eingesetzt.

Das Kohlenwasserstoffgemisch (KG1a) enthält vorzugsweise Cyclohexan, MCP, maximal 0,1 Gew.-% an Aromaten und gegebenenfalls mindestens eine weitere Verbindung ausgewählt aus Olefinen oder nicht-cy-

clischen $C_5$-$C_8$-Alkanen. Besonders bevorzugt enthält das Kohlenwasserstoffgemisch (KG1a) Cyclohexan, Methylcyclopentan (MCP) und mindestens einen weiteren Kohlenwasserstoff, ausgewählt aus Cyclohexan, n-Hexan, iso-Hexanen n-Heptan, iso-Heptanen, Methylcyclohexan oder Dimethylcyclopentanen.

Besonders bevorzugt wird die Ausführungsform A so durchgeführt, dass das Kohlenwasserstoffgemisch (KG1) als Aromat Benzol enthält und/oder das Kohlenwasserstoffgemisch (KG1a) eine gegenüber (KG1) erhöhte Menge an Cyclohexan aufweist.

Die vorstehend beschriebene bevorzugte Ausführungsform A der vorliegenden Erfindung unter Berücksichtigung einer Hydrierung wird in einer bevorzugten Ausgestaltung in Verbindung mit Figur 2 zusätzlich verdeutlicht. In Figur 2 haben die Abkürzungen, Pfeile und sonstigen Symbole eine sinngemäße Bedeutung wie vorstehend für die Figur 1 ausgeführt. Die Hydrierung wird vorzugsweise in mindestens einem Reaktor (HR) durchgeführt, vorzugsweise enthält das Kohlenwasserstoffgemisch (KG1) Benzol, das zu Cyclohexan hydriert wird.

In einer weiteren bevorzugten Ausführungsform des erfindungsgemäßen Verfahrens (nachfolgend auch als "Ausführungsform B" bezeichnet) wird vor Schritt a) aus dem Kohlenwasserstoffgemisch (KG1) mindestens eine Verbindung ausgewählt aus linearen oder verzweigten $C_5$-Alkanen, Cyclopentan oder linearen oder verzweigten $C_6$-Alkanen abgetrennt. Diese Abtrennung, die vorzugsweise destillativ durchgeführt wird, wird nachfolgend auch als "Leichtsiederabtrennung" bezeichnet, die in dem Fachmann bekannten Vorrichtungen, insbesondere unter Verwendung einer Destillationskolonne (D3), durchgeführt werden kann. Unter Leichtsieder versteht man insbesondere $C_5$-$C_6$-Alkane wie Cyclopentan oder iso-Hexane.

Das um die Leichtsieder abgereicherte Kohlenwasserstoffgemisch (KG1) wird anschließend der Isomerisierung, gemäß Schritt a) der vorliegenden Erfindung zugeführt. Das um die Leichtsieder abgereicherte Kohlenwasserstoffgemisch (KG1) wird über eine Abnahmestelle unterhalb des Zulaufs, vorzugsweise aus dem Sumpf der entsprechenden Destillationskolonne, abgetrennt.

Vorzugsweise wird die Leichtsiederabtrennung so durchgeführt, dass vor der Isomerisierung gemäß Schritt a) aus dem Kohlenwasserstoffgemisch (KG1) ein Strom (LS1) enthaltend mindestens eine Verbindung ausgewählt aus linearen oder verzweigten $C_5$-Alkanen, Cyclopentan oder linearen oder verzweigten $C_6$-Alkanen, besonders bevorzugt Isohexane, destillativ abgetrennt wird. Vorzugsweise wird der Strom (LS1) über eine Abnahmestelle oberhalb des Zulaufs, besonders bevorzugt über den Kopf der Destillationskolonne abgezogen.

Bevorzugt ist auch eine Ausführungsform, bei der der aus Schritt b) stammende Strom (LS2) gemäß oben gegebener Beschreibung vollständig oder teilweise in oder vor (D3) zurückgeführt wird.

Sofern vor der Leichtsiederabtrennung eine Hydrierung gemäß Ausführungsform A und/oder eine Schwersieder-Abtrennung gemäß Ausführungsform C (siehe die entsprechenden Textpassagen) durchgeführt wird, wird zur Leichtsiederabtrennung anstelle des Kohlenwasserstoffgemisches (KG1) die entsprechenden Kohlenwasserstoffgemische (KG1 a) bzw. (KG1b) eingesetzt.

Die vorstehend beschriebene bevorzugte Ausführungsform B der vorliegenden Erfindung unter Berücksichtigung einer Leichtsiederabtrennung wird nachfolgend in einer bevorzugten Ausgestaltung in Verbindung mit Figur 3 zusätzlich verdeutlicht. In Figur 3 haben die Abkürzungen, Pfeile und sonstigen Symbole eine sinngemäße Bedeutung wie vorstehend für die Figuren 1 und 2 ausgeführt.

In der Destillationskolonne (D3) werden die Leichtsieder aus dem Kohlenwasserstoffgemisch (KG1) als Strom (LS1) abgetrennt, wobei der Strom (LS1) niedriger siedet als (KG1). Der Strom (LS1) ist gegenüber (KG1) vorzugsweise an Isohexanen und/oder Cyclopentan angereichert und an MCP abgereichert. Das um den Strom (LS1) abgereicherte/verminderte Kohlenwasserstoffgemisch (KG1), das in Figur 3 als "KG1 -(LS1)" bezeichnet wird, siedet höher als (KG1). Der Strom (KG1 -(LS1)) ist gegenüber (KG1) vorzugsweise an Isohexanen und/oder Cyclopentan abgereichert und an MCP angereichert.

Bevorzugt wird die Leichtsiederabtrennung so ausgeführt und betrieben, dass der Strom (LS1) weniger als 5 Gew.-%, besonders bevorzugt weniger als 2,5 Gew.-% MCP enthält und der Strom (KG1 -(LS1)) weniger als 10 Gew.-%, besonders bevorzugt weniger als 5 Gew.-% Isohexane enthält.

Der Strom (LS1) kann beispielsweise in einen Steamcracker als sogenannter Cocrackfeed eingeleitet werden, während der Strom (KG1 -(LS1)) in die Isomerisierungsstufe geführt wird. Gegebenenfalls kann im Rahmen der Leichtsiederabtrennung ein weiterer Strom abgezogen werden, der gegenüber dem Strom (LS) an Isohexanen abgereichert und an leichter als die Isohexane siedenden Komponenten angereichert ist, wie z. B. chlorierte Paraffine mit < 4 Kohlenstoffatomen je Molekül.

In einer weiteren bevorzugten Ausführungsform des erfindungsgemäßen Verfahrens (nachfolgend auch als "Ausführungsform C" bezeichnet) umfasst das Verfahren zusätzlich den Schritt d), der vor Schritt a) und gegebenenfalls vor Schritt c) durchgeführt wird, mit

d) Einspeisung des Kohlenwasserstoffgemisches (KG1) in eine Destillationsvorrichtung (D1), wobei (KG1) mindestens Methylcyclopentan, mindestens ein Alkan mit 7 oder mehr Kohlenstoffatomen und gegebenenfalls einen Aromaten enthält, wobei in (D1) mindestens ein Alkan mit 7 oder mehr Kohlenstoffatomen aus (KG1) unter Erhalt des Kohlenwasserstoffgemisches (KG1b) abgetrennt wird, und

wobei (KG1b) eine gegenüber (KG1) reduzierte Menge an mindestens einem Alkan mit 7 oder mehr Kohlenstoffatomen enthält und in den nachfolgenden Schritten (KG1 b) statt (KG1) verwendet wird.

Vorzugsweise wird in der Destillationsvorrichtung (D1) das im Kohlenwasserstoffgemisch (KG1) enthaltene Alkan mit 7 oder mehr Kohlenstoffatomen (Schwersieder), insbesondere DMP, vollständig oder nahezu vollständig (bis zu 2 % bezogen auf die in (KG1) enthaltene Schwersieder-Menge) aus (KG1), insbesondere vom nichtaromatischen Kohlenwasserstoff und gegebenenfalls vom Aromaten (also den Hauptkomponenten des Gemisches (KG1b)) abgetrennt. Das Alkan mit 7 oder mehr Kohlenstoffatomen wird aus der Destillationsvorrichtung (D1) als Strom (S3) abgezogen, der sich vorzugsweise im Sumpf von (D1) befindet.

Alternativ kann eine nahezu vollständige Schwersieder-Abtrennung, vorzugsweise eine nahezu vollständige DMP-Abtrennung, aus dem Kohlenwasserstoffgemisch (KG1) auch über die im Gemisch (KG1b) verbliebene Schwersieder-Menge, vorzugsweise DMP-Menge, in Bezug auf MCP und/oder Benzol definiert werden. Bei dieser Betrachtungsweise ist es besonders bevorzugt, dass die in der Destillationsvorrichtung (D1) über Kopf als Gemisch (KG1b) abgezogene Schwersieder-Menge, vorzugsweise DMP-Menge, bezogen auf die Summe der über Kopf abgezogenen Mengen an MCP und Benzol höchstens 0,1 Gew.-%, bevorzugt höchstens 0,02 Gew.-% beträgt.

Vorzugsweise ist die Destillationsvorrichtung (D1) eine Rektifikationskolonne. Weiterhin ist es bevorzugt, dass sich der Auslass der Destillationsvorrichtung (D1), aus dem das Gemisch (KG1b) abgetrennt wird, oberhalb des Zulaufs befindet, mit dem das Kohlenwasserstoffgemisch (KG1) in (D1) eingespeist wird, vorzugsweise befindet sich der Auslass im Kopf von (D1).

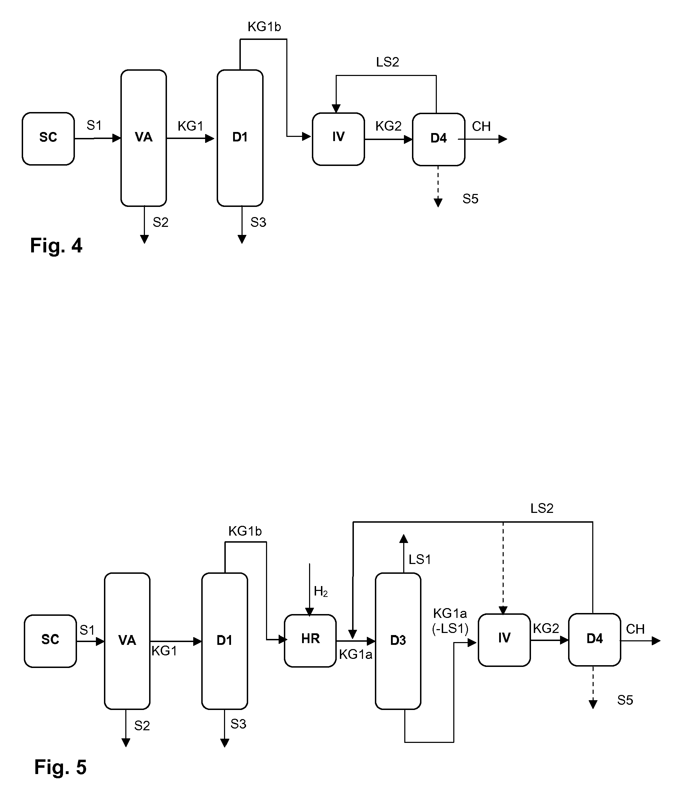

Die vorstehend beschriebene bevorzugte Ausführungsform C der vorliegenden Erfindung unter Berücksichtigung einer Schwersieder-Abtrennung wird in einer bevorzugten Ausgestaltung in Verbindung mit Figur 4 zusätzlich verdeutlicht. In Figur 4 haben die Abkürzungen, Pfeile und sonstigen Symbole eine sinngemäße Bedeutung wie vorstehend für die Figuren 1 bis 3 ausgeführt. Die Schwersieder-Abtrennung wird in einer Destillationsvorrichtung (D1) durchgeführt, die vorzugsweise eine Rektifikationskolonne ist. Vorzugsweise enthält das Kohlenwasserstoffgemisch (KG1) MCP, DMP und gegebenenfalls Benzol. Die Schwersieder, insbesondere DMP, werden vorzugsweise (nahezu) vollständig über den Strom (S3) aus (D1) abgetrennt. Sofern (KG1) auch einen Aromaten, vorzugsweise Benzol, enthält, wird das Kohlenwasserstoffgemisch (KG1b) im Anschluss an die Schwersieder-Abtrennung vorzugsweise einer Hydrierung unterzogen, wie es nachfolgend im Zusammenhang mit der Figur 5 verdeutlicht ist.

Weiterhin ist es in der Ausführungsform C bevorzugt, dass Schritt d) die folgenden Teilschritte aufweist:

    d1) Einspeisung des Kohlenwasserstoffgemisches (KG1) umfassend
    i) Benzol,
    ii) MCP,
    iii) Dimethylpentane (DMP),
    iv) Cyclohexan und
    v) gegebenenfalls mindestens eine weitere Verbindung ausgewählt aus Olefinen oder $C_5$-$C_8$-Alkanen

in die Destillationsvorrichtung (D1),

    d2) Abtrennen eines Stromes (S3) enthaltend DMP aus einem Auslass der Destillationsvorrichtung (D1), wobei sich der Auslass unterhalb des Zulaufs, bevorzugt am Sumpf von (D1) befindet.,

    d3) Abtrennen des Kohlenwasserstoffgemisches (KG1b) enthaltend Benzol und/oder MCP aus einem Auslass der Destillationsvorrichtung (D1), wobei sich der Auslass oberhalb des Zulaufs, bevorzugt am Kopf von (D1) befindet.

In diesem Zusammenhang ist es bevorzugt, dass das Kohlenwasserstoffgemisch (KG1b) mindestens 95%, vorzugsweise mindestens 98% der im Kohlenwasserstoffgemisch (KG1) enthaltenen Teilmenge bestehend aus Benzol und MCP enthält und/oder dass das Kohlenwasserstoffgemisch (KG1b) höchstens 0,1 Gew.%, vorzugsweise höchstens 0,02 Gew.% (bezogen auf die Gesamtmenge an Benzol und MCP in (KG1b)) DMP enthält, besonders bevorzugt enthält das Kohlenwasserstoffgemisch (KG1b) höchstens 0,015 Gew.% (bezogen auf die Gesamtmenge an Benzol und MCP in (KG1b)) 2,4-DMP.

Der aus dem Sumpf der Destillationsvorrichtung (D1) abgetrennte Strom (S3) enthält DMP sowie gegebenenfalls weitere Komponenten. Die weiteren Komponenten sind vorzugsweise Cyclohexan, Schwersieder mit einem Normalsiedepunkt > 78 °C und/oder ungesättigte Verbindungen. Die ungesättigten Verbindungen können teilweise auch als Schwersieder mit einem Normalsiedepunkt > 78 °C aufgefasst werden. Die ungesättigten Verbindungen sind vorzugsweise ausgewählt aus Benzol, Olefinen, cyclischen Olefinen, insbesondere Cyclohexen, Dienen und cyclischen Dienen. Allerdings gilt für Benzol, dass es aufgrund einer Azeotropbildung mit einem Teil der in Strom (KG1 a) enthaltenen Komponenten überwiegend mit dem Strom (KG1a) abgezogen wird.

Vorzugsweise enthält der Strom (S3) mindestens 98 % des DMPs, das im Kohlenwasserstoffgemisch (KG1) enthalten ist, mehr bevorzugt mindestens 99 % des DMPs. Weiterhin ist es bevorzugt, dass der aus dem Sumpf der Destillationsvorrichtung (D1) abgetrennte Strom (S3) höchstens 10 %, bevorzugt höchstens 5 %, besonders bevorzugt höchstens 2 % des in (KG1) enthaltenen MCPs enthält.

Die vorstehend beschriebene bevorzugte Ausführungsform C der vorliegenden Erfindung unter Berücksichtigung einer Schwersieder-Abtrennung in Gegenwart von mindestens einem Aromaten, vorzugsweise Benzol, wird in einer bevorzugten Ausgestaltung in Verbindung mit Figur 5 zusätzlich verdeutlicht. In Figur 5 haben die Abkürzungen, Pfeile und sonstigen Symbole eine sinngemäße Bedeutung wie vorstehend für die Figuren 1 bis 4 ausgeführt. Die Schwersieder-Abtrennung wird in einer Destillationsvorrichtung (D1) durchgeführt, die vorzugsweise eine Rektifikationskolonne ist. Vorzugsweise enthält das Kohlenwasserstoffgemisch (KG1) MCP, DMP, Benzol und gegebenenfalls Leichtsieder. Die Schwersieder, insbesondere DMP, werden vorzugsweise (nahezu) vollständig über den Strom (S3) aus (D1) abgetrennt. Das Kohlenwasserstoffgemisch (KG1b) wird im Anschluss an die Schwersieder-Abtrennung einer Hydrierung unterzogen, wie vorstehend im Zusammenhang mit der Figur 2 verdeutlicht. Die in Figur 5 im Anschluss an die Hydrierung aufgeführte Leichtsieder-Abtrennung in der Destillationskolonne (D3) wird nur dann durchgeführt, sofern in (KG1) Leichtsieder in nennenswertem Umfang vorhanden sind.

In einer weiteren bevorzugten Ausführungsform des erfindungsgemäßen Verfahrens (nachfolgend auch als "Ausführungsform D" bezeichnet), wird der Strom (S3) in eine Destillationsvorrichtung (D2) eingeleitet wird, wobei in (D2) Cyclohexan von DMP abgetrennt wird. Die Ausführungsform D stellt eine spezielle Ausgestaltung der vorstehend beschriebenen Ausführungsform C dar, die durchgeführt werden kann, sofern bei der Schwersieder-Abtrennung gemäß Ausführungsform C aus dem eingesetzten Kohlenwasserstoffgemisches (KG1) DMP und Cyclohexan abgetrennt werden bzw. diese beiden Verbindungen zunächst in (KG1) enthalten sind.

In dieser Ausführungsform D des erfindungsgemäßen Verfahrens wird das im Strom (S3) enthaltene Cyclohexan von den Schwersiedern, insbesondere von DMP abgetrennt, insbesondere durch Destillation in einer Destillationsvorrichtung (D2). Dabei wird der Strom (S3) in die Destillationsvorrichtung (D2) eingeleitet, wobei in (D2) Cyclohexan von DMP abgetrennt wird. Die konkrete Zusammensetzung des Stroms (S3) wurde vorstehend im Zusammenhang mit dem erfindungsgemäßen Schritt d) bereits beschrieben.

Die Destillation bzw. die Destillationsvorrichtung (D2) kann einstufig oder mehrstufig, beispielsweise zweistufig oder dreistufig sein, vorzugsweise ist sie dreistufig. Unter der Anzahl der Stufen (Stufigkeit) wird in diesem Zusammenhang die Anzahl der Kolonnen, jeweils inklusive Nebenapparate wie z. B. Sumpfverdampfer und Kondensatoren, verstanden, die zusammengefasst die Destillationsvorrichtung (D2) ausbilden. Eine dreistufige Destillationsvorrichtung (D2) bedeutet also, dass insgesamt drei Kolonnen, jeweils inklusive Nebenapparate wie z. B. Sumpfverdampfer und Kondensatoren, in denen jeweils ein Destillationsprozess durchgeführt werden kann, gemeinsam die Destillationsvorrichtung (D2) ausbilden. Vorzugsweise umfasst (D2) eine Extraktivdestillationskolonne. Weiterhin ist es bevorzugt, dass der aus (D2) abgezogene an Cyclohexan angereicherte Strom höchstens 0,1 Gew.%, vorzugsweise höchstens 0,02 Gew.% DMP, besonders bevorzugt höchstens 0,015 Gew.% 2,4-DMP enthält.

Sofern die Destillationsvorrichtung (D2) eine Extraktivdestillationskolonne umfasst, erfolgt die Extraktivdestillation bevorzugt unter Verwendung eines Extraktiv-Hilfsmittels (Extraktiv-Hilfsstoffs). Als Extraktiv-Hilfsmittel werden in der Regel Verbindungen eingesetzt, für die die nachfolgende Formel (1) gilt:

$$ \gamma_{DMP,E}^{\infty} \Big/ \gamma_{CH,E}^{\infty} > n \qquad (1) $$

mit

$\gamma_{DMP,E}^{\infty} =$ Aktivitätskoeffizient von 2,4-Dimethylpentan im Extraktiv-Hilfsmittel bei unendlicher Verdünnung,

$\gamma_{CH,E}^{\infty} =$ Aktivitätskoeffizient von Cyclohexan im Extraktiv-Hilfsmittel bei unendlicher Verdünnung,

n = bevorzugt 1,1, besonders bevorzugt 1,3.

Als Extraktiv-Hilfsmittel werden bevorzugt sauerstoffhaltige offenkettige oder cyclische organische Verbindungen mit einem Siedepunkt mindestens 5 K oberhalb desjenigen des Cyclohexans (81 °C) eingesetzt, insbesondere solche, die eine Amid-Funktion R-CO-NR'R" als Strukturelement enthalten, wobei R, R' und R" (unabhängig voneinander) bevorzugt ausgewählt sind aus $C_1$-$C_{30}$-Alkyl oder H. Besonders geeignet als Extraktiv-Hilfsmittel sind N-Methylpyrrolidon, N-Formylmorpholin. Geeignet sind aber auch Verbindungen wie Sulfolan, Dimethylsulfoxid oder andere dem Fachmann als nichtprotische polare Lösungsmittel bekannte Verbindungen. Geeignet sind auch Mischungen mehrerer der genannten Verbindungen untereinander oder mit Wasser. Vorzugsweise umfasst die Cyclohexan/DMP-Trennung die nachfolgenden Schritte i) bis iii) und gegebenenfalls Schritt iv), wobei die Destillationsvorrichtung (D2) durch die drei Komponenten (D2-1) bis (D2-3) ausgebildet wird:

i) eine Rektifizierkolonne (D2-1), in der der Großteil der Schwersieder mit einem Normalsiedepunkt > 84 °C (bezogen auf die Menge im Zulauf zu (D2-1) über Sumpf und der Großteil des Cyclohexans und sonstiger Verbindungen mit einem Normalsiedepunkt von 79 bis 84 °C (bezogen auf die Menge im Zulauf zu D2-1) über Kopf abgetrennt werden,

ii) eine Extraktivdestillationskolonne (D2-2), in der das Kopfprodukt aus (D2-1) mit einem Extraktiv-Hilfsmittel zusammengeführt und derart destilliert wird, dass der Großteil des Extraktiv-Hilfsmittels und des Cyclohexans über Sumpf und der Großteil der im Kopfprodukt aus (D2-1) enthaltenen sonstigen Verbindungen mit einem Normalsiedepunkt von 79 bis 84 °C über Kopf aus (D2-2) abgezogen werden,

iii) eine Regenerationskolonne (D2-3), in der der Großteil des im Sumpfstrom aus (D2-2) enthaltenen Cyclohexans über Kopf und der Großteil des im Sumpfstrom aus (D2-2) enthaltenen Extraktiv-Hilfsmittel über Sumpf abgezogen werden und

iv) gegebenenfalls eine Hydrierungsvorrichtung, in die entweder der Strom (S3) oder das Kopfprodukt aus (D2-3) geführt wird.

Im Rahmen der vorstehenden Schritte i) bis iv) bedeutet die Angabe "über Sumpf eine Abnahmestelle unterhalb des Zulaufs, bevorzugt den Sumpf und die Angabe "über Kopf" eine Abnahmestelle oberhalb des Zulaufs, bevorzugt den Kopf der jeweiligen Kolonne.

Der in der vorstehenden Ausführungsform enthaltene optionale Schritt iv) wird in der Regel nur dann durchgeführt, wenn im Strom (S3) ungesättigte Verbindungen enthalten sind, die somit auch in die Destillationsvorrichtung (D2) eingespeist werden und die zudem nicht über den Sumpf der Rektifizierkolonne (D2-1) aus dem Verfahren ausgeschleust werden. Die Hydrierung gemäß optionalem Schritt iv) kann sinngemäß zu der Hydrierung gemäß der vorstehend beschriebenen Ausführungsform A durchgeführt werden, bevorzugt erfolgt sie einstufig. Die Hydrierungsvorrichtung kann gegebenenfalls auch der Destillationsvorrichtung (D2) vorgeschaltet sein. In diesem Fall wird der Strom (S3) zunächst in die Hydrierungsvorrichtung geführt, anschließend wird der hydrierte Strom (S3) in die Destillationsvorrichtung (D2), insbesondere in die Rektifizierkolonne (D2-1), eingeleitet. Dies ist eine vorteilhafte Variante, wenn in Strom (S3) Komponenten wie z.B. ungesättigte Kohlenwasserstoffe enthalten sind, die mit den über den Sumpf von (D2-1) zu ziehenden Komponenten Azeotrope bilden.

Gegebenenfalls kann die vorstehend beschriebene bevorzugte Ausführungsform der Cyclohexan-/DMP-Trennung auch ohne Rektifizierkolonne (D2-1) als zwingender Bestandteil durchgeführt werden. Bei dieser Variante erfolgt die Cyclohexan-/DMP-Trennung nur unter Verwendung der beiden Kolonnen (D2-2) und (D2-3) sinngemäß, wobei gegebenenfalls auch eine Hydrierungsvorrichtung nachgeschaltet sein kann. Diese Variante wird vorzugsweise dann durchgeführt, wenn im Strom (S3) kein oder nur ein geringer Anteil an Schwersiedern mit einem Normalsiedepunkt > 84 °C enthalten ist.

Die vorstehend beschriebene bevorzugte Ausführungsform unter Verwendung der Extraktivdestillationskolonne (D2-2) wird bevorzugt so ausgeführt und betrieben, dass der über Kopf aus (D2-2) abgezogen DMP-haltige Strom weniger als 50 Gew.-%, bevorzugt weniger als 10 Gew.-% Cyclohexan enthält. Weiterhin enthält der über Kopf Regenerationskolonne (D2-3) abgezogene cyclohexanhaltige Strom vorzugsweise weniger als 1 Gew.-%, mehr bevorzugt weniger als 10 Gew.-ppm Extraktiv-Hilfsmittel und/oder weniger als 1 Gew.-%, bevorzugt weniger als 300 Gew.-ppm Dimethylpentane, besonders bevorzugt weniger als 150 Gew.-ppm 2,4-Dimethylpentan.

Weiterhin ist es bevorzugt, dass Cyclohexan in einer Reinheit von 98 Gew.-%, insbesondere mindestens 99,5 Gew.-%, aus (D2) isoliert wird. Bezüglich der Durchführung der Isolierung des Cyclohexans gelten die gleichen Überlegungen wie vorstehend im Zusammenhang mit der Isolierung des Cyclohexan gemäß Schritt b), insbesondere im Zusammenhang mit der Destillationsvorrichtung (D4) ausgeführt. Alternativ kann das Cyclohexan, das aus der Destillationsvorrichtung (D2) gemäß der Ausführungsform D stammt, mit dem Cyclohexan, das bei der Isomerisierung gemäß Schritt a) und/oder gegebenenfalls bei der Hydrierung gemäß Schritt c) hergestellt wurde, zusammengeführt wird.

Die vorstehend beschriebene Ausführungsform D der vorliegenden Erfindung wird in einer bevorzugten Ausgestaltung in Verbindung mit Figur 6 zusätzlich verdeutlicht. In Figur 6 haben die Abkürzungen, Pfeile und sonstigen Symbole eine sinngemäße Bedeutung, die vorstehend für Figur 1 beziehungsweise in der Beschreibung dieser bevorzugten Ausführungsform ausgeführt ist. In der Ausführungsform gemäß Figur 6 wird eine Destillationsvorrichtung (D2), bestehend im Wesentlichen aus drei Kolonnen ((D2-1) bis D2-3)), eingesetzt, der eine Hydrierungsvorrichtung nachgeschaltet ist. Die einzelnen Kolonnen können weiterhin auch Nebenapparate wie Sumpfverdampfer oder Kondensatoren umfassen, die der Übersichtlichkeit halber in Figur 6 nicht wiedergegeben sind. HV bedeutet Hydrierungsvorrichtung, EHM bedeutet Extraktiv-Hilfsmittel, S > 84 bedeutet Schwersieder mit einem Normalsiedepunkt > 84 °C, 24DMP bedeutet 2,4-Dimethylpentan und die in Klammer gesetzten Ausdrücke geben die für das Verfahren relevantesten und/oder die Hauptkomponenten des jeweiligen Stroms an. 24DMP ist beispielhaft als bevorzugte Komponente der (sonstigen) Verbindungen mit einem Normalsiedepunkt von 79 bis 84 °C aufgeführt. Als Extraktiv-Hilfsmittel wird vorzugsweise N-Methyl-2-pyrrolidon (NMP) verwendet.

Der bevorzugt aus dem Sumpf der Destillationsvorrichtung (D1) stammende Strom (S3), der DMP, Cyclohexan, gegebenenfalls ungesättigte Verbindungen und gegebenenfalls Schwersieder mit einem Normalsiedepunkt > 84 °C enthält, wird, gegebenenfalls nach einer Hydrierung, in die Rektifizierkolonne (D2-1) eingespeist. Die ungesättigten Verbindungen sind vorzugsweise ausgewählt aus Benzol, Olefinen, cyclischen Olefinen, insbesondere Cyclohexen, Dienen und cyclischen Dienen. In (D2-1) erfolgt die Aufkonzentrierung des im Strom (S1) enthaltenen Cyclohexans, wobei der Strom (S3) zunächst mittels Rektifikation in einen an schwerer als Cyclohexan siedenden Komponenten (also beispielsweise 3,3-DMP sowie sonstige Schwersieder mit einem Normalsiedepunkt > 84 °C oder entsprechend siedende ungesättigte Verbindungen) angereicherten Strom 15 und einen an schwerer als Cyclohexan siedenden Komponenten abgereicherten Strom 16 (Strom 16 enthält also Cyclohexan sowie einen Großteil der sonstigen Verbindungen mit einem Siedepunkt von 79 bis 84 °C, zumindest eine Teilmenge der ungesättigten Verbindungen und eine Restmenge an Schwersiedern mit einem Normalsiedepunkt > 84 °C) aufgetrennt wird. Strom 15 kann beispielsweise als Cofeed zu einem Steamcracking-Prozess geführt oder als Bestandteil von Kraftstoff-Mischungen verwendet werden.

Strom 16 wird in eine Extraktivdestillationskolonne (D2-2) geführt. In die Extraktivdestillationskolonne (D2-2) wird an einer Stelle oberhalb des Zulaufs von Strom 16 ein mindestens ein Extraktiv-Hilfsmittel (EHM) enthaltender Strom 17 geführt. An einer Stelle ebenfalls oberhalb des Zulaufs von Strom 16, bevorzugt oberhalb des Zulaufs von Strom 17, z. B. am Kolonnenkopf bzw. nach dem Kopfkondensator der Kolonne, wird ein Strom 18 entnommen, der gegenüber Strom 16 an DMP, insbesondere an 2,4-DMP, angereichert ist. Vorzugsweise enthält der Strom 18 einen Großteil der im Strom 16 enthaltenen sonstigen Verbindungen mit einem Normalsiedepunkt von 79 bis 84 °C, insbesondere an 2,4-DMP. Über eine Stelle unterhalb des Zulaufs von Strom 16, bevorzugt über den Kolonnensumpf, wird ein Strom 19 entnommen, enthaltend das Extraktiv-Hilfsmittel, Cyclohexan und die ungesättigten Verbindungen, wobei in Strom 19 das Konzentrationsverhältnis Cyclohexan/ DMP, insbesondere an Cyclohexan/2,4-DMP höher ist als im Strom 16.

Die Extraktivdestillationskolonne (D2-2) wird vorzugsweise so ausgeführt und betrieben, dass Strom 18 höchstens 100 Gew.-ppm, bevorzugt höchstens 10 Gew.-ppm, besonders bevorzugt höchstens 1 Gew.-ppm an Extraktiv-Hilfsmittel enthält. Dies kann erreicht werden, indem der höchste Zulauf eines EHM-haltigen Stroms mindestens 5, bevorzugt mindestens 10 theoretische Trennstufen (gemäß dem Fachmann bekannter Definition) unterhalb der Abnahmestelle von Strom 18 erfolgt und/oder (D2-2) mit einem Rücklaufverhältnis von mindestens 5, bevorzugt mindestens 10 betrieben wird.

Strom 19 wird, gegebenenfalls nach einer Vorwärmung, in die Regenerationskolonne (D2-3) geführt. Aus der Regenerationskolonne (D2-3) wird ein gegenüber Strom 19 an Cyclohexan angereicherter Strom 20 und ein gegenüber Strom 19 an Cyclohexan abgereicherter Strom 21 (Strom 21 enthält vordergründig das Extraktiv-Hilfsmittel, eine Teilmenge an Cyclohexan und (gegebenenfalls) eine Restmenge an sonstigen Verbindungen mit einem Normalsiedepunkt von 79 bis 84 °C, insbesondere an 2,4-DMP) abgezogen. Aus Strom 21 wird ein Ausschleusstrom (Purgestrom) 21 a abgezweigt, der bevorzugt maximal 5 %, besonders bevorzugt maximal 1 % der Menge von Strom 21 ausmacht. Der verbleibende Strom wird, gegebenenfalls nach einer Abkühlung (die auch im Wärmeverbund mit einer Vorwärmung von Strom 19 erfolgen kann), mindestens teilweise dem Strom 17 zugeführt und/oder in der Nähe des Stroms 16 in die Extraktivdestillationskolonne (D2-2) zurückgeführt.

Strom 20 wird gegebenenfalls, zusammen mit einem Wasserstoff enthaltenden Strom, in die Hydrierungsvorrichtung (HV) geführt, in der mit Hilfe eines geeigneten Katalysators die ungesättigten Verbindungen, ausgewählt aus Benzol, Olefinen, cyclischen Olefinen, insbesondere Cyclohexen, Dienen und cyclischen Dienen, hydriert werden. Wasserstoff kann aber auch getrennt von Strom 20 in (HV) eingeleitet werden, wie in Figur 6 dargestellt. Der bei der Hydrierung gewonnene Strom 22 enthält als Hauptbestandteil Cyclohexan und kann gegebenenfalls weiter aufgearbeitet werden. beispielsweise kann spezifikationsgerechtes (hochreines) Cyclohexan aus dem Strom 22 isoliert werden. Gegebenenfalls kann der Strom 22 auch mit dem Cyclohexan oder einem cyclohexanhaltigen Strom vereinigt werden, der im erfindungsgemäßen Verfahren in der Vorrichtung (HR) und/oder der Vorrichtung (IV) (gemäß der Grundform und/oder den Ausführungsformen A bis C) hergestellt wird.

Sofern im Kohlenwasserstoffgemisch (KG1) auch ein Aromat enthalten ist, ist dieser vorzugsweise Benzol. Im Rahmen der vorliegenden Erfindung wird weiterhin vorzugsweise eine Kombination der vorstehend beschriebenen Ausführungsformen A bis C durchgeführt, so wie sie beispielhaft in Figur 5 dargestellt ist, vorzugsweise in Kombination mit den bevorzugten Ausführungsformen gemäß Schritt b), wie sie beispielhaft in den Figuren 7 und 8 dargestellt sind. Weiterhin ist eine zusätzliche Kombination mit der Ausführungsform D bevorzugt.

**Patentansprüche**

1.  Verfahren zur Herstellung von Cyclohexan umfassend die folgenden Schritte

    a) Isomerisierung eines Kohlenwasserstoffgemisches (KG1) enthaltend Methylcyclopentan (MCP) in Gegenwart eines Katalysators unter Erhalt eines Kohlenwasserstoffgemisches (KG2) enthaltend Cyclohexan,
    wobei (KG1) in einer Vorrichtung zur Aromatenabtrennung, die einem Steamcrackverfahren nachgeschaltet ist, aus einem aus dem Steamcrackverfahren stammenden Strom (S1) gewonnen wird, und
    b) Isolierung von Cyclohexan aus dem Kohlenwasserstoffgemisch (KG2).

2.  Verfahren gemäß Anspruch 1, **dadurch gekennzeichnet, dass** die Aromatenabtrennung eine Aromaten-Extraktivdestillation, vorzugsweise eine Benzol-Extraktivdestillation, ist und/oder das Kohlenwasserstoffgemisch (KG1) eine geringere Konzentration an Aromaten aufweist als der Zustrom (S1) zur Vorrichtung zur Aromatenabtrennung.

3. Verfahren gemäß Anspruch 1 oder 2, umfassend zusätzlich den Schritt c), der vor Schritt a) durchgeführt wird, mit

  c) Hydrierung des Kohlenwasserstoffgemisches (KG1) enthaltend Methylcyclopentan (MCP) und mindestens einen Aromaten, unter Erhalt eines Kohlenwasserstoffgemisches (KG1a), das eine gegenüber (KG1) reduzierte Menge an mindestens einem Aromaten aufweist, wobei in den nachfolgenden Schritten (KG1 a) statt (KG1) verwendet wird.

4. Verfahren gemäß Anspruch 3, **dadurch gekennzeichnet, dass** das Kohlenwasserstoffgemisch (KG1) als Aromat Benzol enthält und/oder das Kohlenwasserstoffgemisch (KG1a) eine gegenüber (KG1) erhöhte Menge an Cyclohexan aufweist.

5. Verfahren gemäß Anspruch 3 oder 4, **dadurch gekennzeichnet, dass** die Hydrierung des Kohlenwasserstoffgemisches (KG1) in Gegenwart eines Katalysators durchgeführt wird, der als Aktivmetall mindestens ein Element der 8. bis 10. Gruppe des Periodensystems der Elemente enthält, insbesondere Nickel oder Ruthenium.

6. Verfahren gemäß einem der Ansprüche 3 bis 5, **dadurch gekennzeichnet, dass** das Kohlenwasserstoffgemisch (KG1) Benzol, Methylcyclopentan (MCP) und mindestens eine weitere Verbindung ausgewählt aus Cyclohexan, Cyclopentan, Olefinen oder nicht-cyclischen $C_5$-$C_8$-Alkanen enthält.

7. Verfahren gemäß einem der Ansprüche 1 bis 6, **dadurch gekennzeichnet, dass** in Schritt a) der Katalysator eine saure ionische Flüssigkeit ist, vorzugsweise enthält die saure ionische Flüssigkeit als Kation ein zumindest teilweise alkyliertes Ammoniumion oder ein heterocyclisches Kation und/oder als Anion ein Chloroaluminat mit der Zusammensetzung $Al_nCl_{(3n+1)}$ mit $1<n<2,5$.

8. Verfahren gemäß einem der Ansprüche 1 bis 7, **dadurch gekennzeichnet, dass** vor Schritt a) aus dem Kohlenwasserstoffgemisch (KG1) mindestens eine Verbindung ausgewählt aus linearen oder verzweigten $C_5$-Alkanen, Cyclopentan oder linearen oder verzweigten $C_6$-Alkanen destillativ abgetrennt wird.

9. Verfahren gemäß einem der Ansprüche 1 bis 8 umfassend zusätzlich den Schritt d), der vor Schritt a) und gegebenenfalls vor Schritt c) durchgeführt wird, mit

  d) Einspeisung des Kohlenwasserstoffgemisches (KG1) in eine Destillationsvorrichtung (D1),
  wobei (KG1) Methylcyclopentan (MCP), mindestens ein Alkan mit 7 oder mehr Kohlenstoffatomen und gegebenenfalls einen Aromaten enthält,
  wobei in (D1) mindestens ein Alkan mit 7 oder mehr Kohlenstoffatomen aus (KG1) unter Erhalt des Kohlenwasserstoffgemisches (KG1b) abgetrennt wird, und
  wobei (KG1b) eine gegenüber (KG1) reduzierte Menge an mindestens einem Alkan mit 7 oder mehr Kohlenstoffatomen enthält und in den nachfolgenden Schritten (KG1 b) statt (KG1) verwendet wird.

10. Verfahren gemäß Anspruch 9, **dadurch gekennzeichnet, dass** Schritt d) die folgenden Teilschritte aufweist:

  d1) Einspeisung des Kohlenwasserstoffgemisches (KG1) umfassend

   i) Benzol,
   ii) MCP,
   iii) Dimethylpentane (DMP),
   iv) Cyclohexan und
   v) gegebenenfalls mindestens eine weitere Verbindung ausgewählt aus Olefinen oder $C_5$-$C_8$-Alkanen

  in die Destillationsvorrichtung (D1),
  d2) Abtrennen eines Stromes (S3) enthaltend DMP aus einem Auslass der Destillationsvorrichtung (D1), wobei sich der Auslass unterhalb des Zulaufs, bevorzugt am Sumpf von (D1) befindet,
  d3) Abtrennen des Kohlenwasserstoffgemisches (KG1b) enthaltend Benzol und/oder MCP aus einem Auslass der Destillationsvorrichtung (D1), wobei sich der Auslass oberhalb des Zulaufs, bevorzugt am Kopf von (D1) befindet.

11. Verfahren gemäß Anspruch 10, **dadurch gekennzeichnet, dass** das Kohlenwasserstoffgemisch (KG1b) mindestens 95%, vorzugsweise mindestens 98% der im Kohlenwasserstoffgemisch (KG1) enthaltenen Teilmenge bestehend aus Benzol und MCP enthält und/oder dass das Kohlenwasserstoffgemisch (KG1b) höchstens 0,1 Gew.%, vorzugsweise höchstens 0,02 Gew.% (bezogen auf die Gesamtmenge an Benzol und MCP in (KG1b)) DMP enthält, besonders bevorzugt enthält das Kohlenwasserstoffgemisch (KG1b) höchstens 0,015 Gew.% (bezogen auf die Gesamtmenge an Benzol und MCP in (KG1b)) 2,4-DMP.

**12.** Verfahren gemäß einem der Ansprüche 9 bis 11, **dadurch gekennzeichnet, dass** der Strom (S3) in eine Destillationsvorrichtung (D2) eingeleitet wird, wobei in (D2) Cyclohexan von DMP abgetrennt wird, vorzugsweise umfasst (D2) eine Extraktivdestillationskolonne und/oder vorzugsweise enthält der aus (D2) abgezogene an Cyclohexan angereicherte Strom höchstens 0,1 Gew.%, vorzugsweise höchstens 0,02 Gew.% DMP, besonders bevorzugt höchstens 0,015 Gew.% 2,4-DMP.

**13.** Verfahren gemäß Anspruch 12, **dadurch gekennzeichnet, dass** die Cyclohexan-/DMP-Trennung die nachfolgenden Schritte i) bis iii) und gegebenenfalls Schritt iv) umfasst, wobei die Destillationsvorrichtung (D2) durch die drei Komponenten (D2-1) bis (D2-3) ausgebildet wird:

　　i) eine Rektifizierkolonne (D2-1), in der der Großteil der Schwersieder mit einem Normalsiedepunkt > 84 °C (bezogen auf die Menge im Zulauf zu (D2-1)) über Sumpf und der Großteil des Cyclohexans und sonstige Verbindungen mit einem Normalsiedepunkt von 79 bis 84 °C (bezogen auf die Menge im Zulauf zu D2-1) über Kopf abgetrennt werden,
　　ii) eine Extraktivdestillationskolonne (D2-2), in der das Kopfprodukt aus (D2-1) mit einem Extraktiv-Hilfsmittel zusammengeführt und derart destilliert wird, dass der Großteil des Extraktiv-Hilfsmittels und des Cyclohexans über Sumpf und der Großteil der im Kopfprodukt aus (D2-1) enthaltenen sonstigen Verbindungen mit einem Normalsiedepunkt von 79 bis 84 °C über Kopf aus (D2-2) abgezogen werden,
　　iii) eine Regenerationskolonne (D2-3), in der der Großteil des im Sumpfstrom aus (D2-2) enthaltenen Cyclohexans über Kopf und der Großteil des im Sumpfstrom aus (D2-2) enthaltenen Extraktiv-Hilfsmittel über Sumpf abgezogen werden und
　　iv) gegebenenfalls eine Hydrierungsvorrichtung, in die entweder der Strom (S3) oder das Kopfprodukt aus (D2-3) geführt wird.

**14.** Verfahren gemäß Anspruch 13, **dadurch gekennzeichnet, dass** Cyclohexan, das aus der Destillationsvorrichtung (D2) stammt, mit dem Cyclohexan, das bei der Isomerisierung gemäß Schritt a) und gegebenenfalls bei der Hydrierung gemäß Schritt c) hergestellt wurde, zusammengeführt wird.

**15.** Verfahren gemäß einem der Ansprüche 1 bis 14, **dadurch gekennzeichnet, dass** in Schritt b) Cyclohexan in einer Reinheit von mindestens 98 Gew.-%, vorzugsweise von mindestens 99,5 Gew. %, besonders bevorzugt von mindestens 99,9 Gew.-% isoliert wird.

**16.** Verfahren gemäß einem der Ansprüche 1 bis 15, **dadurch gekennzeichnet, dass** das Kohlenwasserstoffgemisch (KG2) enthaltend Cyclohexan, MCP, gegebenenfalls nicht-cyclische $C_5$-$C_6$-Alkane und gegebenenfalls schwerer als Cyclohexan siedende Komponenten in eine Destillationskolonne (D4) eingespeist wird, wobei aus (D4) an einer Entnahmestelle oberhalb des Zulaufs, bevorzugt über Kopf, der Großteil des im Zulauf zu (D4) enthaltenen MCP und gegebenenfalls an nicht-cyclischen $C_5$-$C_6$-Alkanen abgetrennt und in oder vor die Isomerisierung gemäß Schritt a) zurückgeführt wird.

**17.** Verfahren gemäß Anspruch 16, **dadurch gekennzeichnet, dass** aus der Destillationskolonne (D4) Cyclohexan in einer Reinheit von mindestens 98 Gew.-% über den Sumpf von (D4) oder über einen unterhalb des Zulaufs gelegenen Seitenabzug von (D4), vorzugsweise über einen dampfförmigen Seitenabzug von (D4), abgezogen wird.

**18.** Verfahren gemäß Anspruch 17, **dadurch gekennzeichnet, dass** der über den Sumpf von (D4) abgezogene an Cyclohexan angereicherte Strom in eine Destillationskolonne (D5) eingeleitet wird, wobei über den Sumpf von (D5) ein Strom (S5), enthaltend schwerer als Cyclohexan siedende Komponenten, abgetrennt wird und über eine Abnahmestelle oberhalb des Zulaufs zu (D5), bevorzugt über Kopf, Cyclohexan mit einer Reinheit von mindestens 98 Gew.-%, vorzugsweise von mindestens 99,5 Gew. %, besonders bevorzugt von mindestens 99,9 Gew.-% abgezogen wird.

**19.** Verfahren gemäß Anspruch 17, **dadurch gekennzeichnet, dass** ein an Cyclohexan angereicherter Strom über den Seitenabzug aus der Destillationskolonne (D4) abgetrennt wird, wobei sich der Seitenabzug vorzugsweise im Abtriebsteil von (D4) befindet und/oder der an Cyclohexan angereicherte Strom aus dem Seitenabzug von (D4) in eine bevorzugt als Destillationskolonne ausgeführte Vorrichtung (D6) zur weiteren Aufreinigung geleitet wird und dort über eine Abnahmesstelle oberhalb des Zulaufs von (D6), bevorzugt über Kopf, Cyclohexan mit einer Reinheit von mindestens 98 Gew.-%, vorzugsweise von mindestens 99,5 Gew. %, besonders bevorzugt von mindestens 99,9 Gew.-% gewonnen wird.

**20.** Verfahren gemäß einem der Ansprüche 16 bis 19, **dadurch gekennzeichnet, dass** die Destillationskolonne (D4) als Trennwandkolonne ausgeführt ist, die Trennwand sich teilweise unterhalb der Zulaufstelle befindet, eine Abzugsstelle im Bereich der Trennwand liegt und über diese Abzugsstelle ein bevorzugt flüssiger Cyclohexan-Strom mit einer Reinheit von mindestens 98 Gew.-%, vorzugsweise von mindestens 99,5 Gew. %, besonders bevorzugt von mindestens 99,9 Gew.-% entnommen wird.

**Claims**

1. A process for preparing cyclohexane, comprising the following steps:

   a) isomerizing a hydrocarbon mixture (HM1) comprising methylcyclopentane (MCP) in the presence of a catalyst to obtain a hydrocarbon mixture (HM2) comprising cyclohexane, (HM1) being obtained in an apparatus for aromatics removal connected downstream of a steamcracking process, from a stream (S1) originating from the steamcracking process, and
   b) isolating cyclohexane from the hydrocarbon mixture (HM2).

2. The process according to claim 1, wherein the aromatics removal is an extractive aromatics distillation, preferably an extractive benzene distillation, and/or the hydrocarbon mixture (HM1) has a lower concentration of aromatics than the feed stream (S1) to the apparatus for aromatics removal.

3. The process according to claim 1 or 2, additionally comprising step c), which is performed prior to step a), comprising

   c) hydrogenating the hydrocarbon mixture (HM1) comprising methylcyclopentane (MCP) and at least one aromatic to obtain a hydrocarbon mixture (HM1a) having a reduced amount of at least one aromatic compared to (HM1), with use of (HM1a) rather than (HM1) in the subsequent steps.

4. The process according to claim 3, wherein the aromatic present in the hydrocarbon mixture (HM1) is benzene and/or the hydrocarbon mixture (HM1a) comprises an increased amount of cyclohexane compared to (HM1).

5. The process according to claim 3 or 4, wherein the hydrogenation of the hydrocarbon mixture (HM1) is performed in the presence of a catalyst comprising, as an active metal, at least one element of groups 8 to 10 of the Periodic Table of the Elements, especially nickel or ruthenium.

6. The process according to any of claims 3 to 5, wherein the hydrocarbon mixture (HM1) comprises benzene, methylcyclopentane (MCP) and at least one further compound selected from cyclohexane, cyclopentane, olefins and acyclic $C_5$-$C_8$-alkanes.

7. The process according to any of claims 1 to 6, wherein the catalyst used in step a) is an acidic ionic liquid, the acidic ionic liquid preferably comprising, as a cation, an at least partly alkylated ammonium ion or a heterocyclic cation and/or, as an anion, a chloroaluminate ion having the composition $Al_nCl_{(3n+1)}$ where $1 < n < 2.5$.

8. The process according to any of claims 1 to 7, wherein step a) is preceded by distillative separation of at least one compound selected from linear or branched $C_5$-alkanes, cyclopentane and linear or branched $C_6$-alkanes from the hydrocarbon mixture (HM1).

9. The process according to any of claims 1 to 8, additionally comprising step d), which is performed prior to step a) and optionally prior to step c), comprising

   d) feeding the hydrocarbon mixture (HM1) into a distillation apparatus (D1),

   (HM1) comprising methylcyclopentane (MCP), at least one alkane having 7 or more carbon atoms and possibly an aromatic, at least one alkane having 7 or more carbon atoms being removed in (D1) from (HM1) to obtain the hydrocarbon mixture (HM1b), and (HM1b) comprising a reduced amount of at least one alkane having 7 or more carbon atoms compared to (HM1), and (HM1b) being used rather than (HM1) in the subsequent steps.

10. The process according to claim 9, wherein step d) has the following component steps:

    d1) feeding the hydrocarbon mixture (HM1) comprising

       i) benzene,
       ii) MCP,
       iii) dimethylpentanes (DMP),
       iv) cyclohexane and
       v) optionally at least one further compound selected from olefins and $C_5$-$C_8$-alkanes into the distillation apparatus (D1),

    d2) removing a stream (S3) comprising DMP from an outlet of the distillation apparatus (D1), the outlet being below the feed, preferably at the bottom of (D1),
    d3) removing the hydrocarbon mixture (HM1b) comprising benzene and/or MCP from an outlet of the distillation apparatus (D1), the outlet being above the feed, preferably at the top of (D1).

**11.** The process according to claim 10, wherein the hydrocarbon mixture (HM1b) comprises at least 95%, preferably at least 98%, of the portion consisting of benzene and MCP present in the hydrocarbon mixture (HM1), and/or the hydrocarbon mixture (HM1b) comprises at most 0.1% by weight, preferably at most 0.02% by weight (based on the total amount of benzene and MCP in (HM1b)), of DMP, the hydrocarbon mixture (HM1b) more preferably comprising at most 0.015% by weight (based on the total amount of benzene and MCP in (HM1b)) of 2,4-DMP.

**12.** The process according to any of claims 9 to 11, wherein stream (S3) is introduced into a distillation apparatus (D2), cyclohexane being separated from DMP in (D2), and (D2) preferably comprising an extractive distillation column and/or the cyclohexane-enriched stream drawn off from (D2) preferably comprising at most 0.1% by weight, preferably at most 0.02% by weight, of DMP, more preferably at most 0.015% by weight of 2,4-DMP.

**13.** The process according to claim 12, wherein the cyclohexane/DMP separation comprises the following steps i) to iii) and optionally step iv), the distillation apparatus (D2) being formed by the three components (D2-1) to (D2-3) :

 i) a rectifying column (D2-1) in which the majority of the high boilers having a standard boiling point > 84°C (based on the amount in the feed to (D2-1)) is removed via the bottom and the majority of the cyclohexane and other compounds having a standard boiling point of 79 to 84°C (based on the amount in the feed to D2-1) is removed via the top,
 ii) an extractive distillation column (D2-2) in which the top product from (D2-1) is combined with an extraction aid and distilled in such a way that the majority of the extraction aid and of the cyclohexane is drawn off via the bottom and the majority of the other compounds having a standard boiling point of 79 to 84°C present in the top product from (D2-1) is drawn off from (D2-2) via the top,
 iii) a regeneration column (D2-3) in which the majority of the cyclohexane present in the bottom stream from (D2-2) is drawn off via the top and the majority of the extraction aid present in the bottom stream from (D2-2) is drawn off via the bottom, and
 iv) optionally a hydrogenation apparatus into which either stream (S3) or the top product from (D2-3) is conducted.

**14.** The process according to claim 13, wherein cyclohexane which originates from the distillation apparatus (D2) is combined with the cyclohexane which has been prepared in the isomerization in step a) and possibly in the hydrogenation in step c).

**15.** The process according to any of claims 1 to 14, wherein, in step b), cyclohexane is isolated in a purity of at least 98% by weight, preferably of at least 99.5% by weight, more preferably of at least 99.9% by weight.

**16.** The process according to any of claims 1 to 15, wherein the hydrocarbon mixture (HM2) comprising cyclohexane, MCP, possibly acyclic $C_5$-$C_6$-alkanes and possibly higher-boiling components than cyclohexane is fed into a distillation column (D4), and the majority of the MCP and, if present, of acyclic $C_5$-$C_6$-alkanes present in the feed to (D4) is removed from (D4) at a withdrawal point above the feed, preferably via the top, and recycled into or upstream of the isomerization in step a).

**17.** The process according to claim 16, wherein cyclohexane is drawn off from the distillation column (D4) in a purity of at least 98% by weight via the bottom of (D4) or via a side draw from (D4) below the feed, preferably via a vaporous side draw from (D4).

**18.** The process according to claim 17, wherein the cyclohexane-enriched stream drawn off via the bottom of (D4) is introduced into a distillation column (D5), and a stream (S5) comprising higher-boiling components than cyclohexane is removed via the bottom of (D5) and cyclohexane is drawn off with a purity of at least 98% by weight, preferably of at least 99.5% by weight, more preferably of at least 99.9% by weight, via a takeoff point above the feed to (D5), preferably via the top.

**19.** The process according to claim 17, wherein a cyclohexane-enriched stream is removed via the side draw from the distillation column (D4), the side draw preferably being in the stripping section of (D4) and/or the cyclohexane-enriched stream from the side draw of (D4) being passed into an apparatus (D6) for further purification, preferably in the form of a distillation column, and cyclohexane being obtained therein via a takeoff point above the feed of (D6), preferably via the top, with a purity of at least 98% by weight, preferably of at least 99.5% by weight, more preferably of at least 99.9% by weight.

**20.** The process according to any of claims 16 to 19, wherein the distillation column (D4) takes the form of a dividing wall column, the dividing wall is partly below the feed point, a draw point is in the region of the dividing wall and this draw point is used to withdraw a preferably liquid cyclohexane stream having a purity of at least 98% by weight, preferably of at least 99.5% by weight, more preferably of at least 99.9% by weight.

## Revendications

1.  Procédé pour la production de cyclohexane, comprenant les étapes suivantes, consistant à :

    a) isomériser un mélange hydrocarboné (KG1) contenant du méthylcyclopentane (MCP) en présence d'un catalyseur avec obtention d'un mélange hydrocarboné (KG2) contenant du cyclohexane,
    (KG1) étant obtenu dans un dispositif pour la séparation d'aromatiques, qui est disposé en aval d'un procédé de craquage à la vapeur, à partir d'un flux (S1) provenant du procédé de craquage à la vapeur, et
    b) isoler le cyclohexane du mélange hydrocarboné (KG2).

2.  Procédé selon la revendication 1, **caractérisé en ce que** la séparation des aromatiques est une distillation extractive des aromatiques, de préférence une distillation extractive de benzène et/ou le mélange hydrocarboné (KG1) présente une concentration inférieure en aromatiques que le flux d'alimentation (S1) vers le dispositif de séparation des aromatiques.

3.  Procédé selon la revendication 1 ou 2, comprenant en outre l'étape c), qui est réalisée avant l'étape a), consistant à

    c) hydrogéner le mélange hydrocarboné (KG1) contenant du méthylcyclopentane (MCP) et au moins un aromatique avec obtention d'un mélange hydrocarboné (KG1a), qui présente une quantité réduite d'au moins un aromatique par rapport à (KG1), (KG1a) étant utilisé à la place de (KG1) dans les étapes suivantes.

4.  Procédé selon la revendication 3, **caractérisé en ce que** le mélange hydrocarboné (KG1) contient du benzène comme aromatique et/ou le mélange hydrocarboné (KG1a) présente une quantité augmentée de cyclohexane par rapport à (KG1).

5.  Procédé selon la revendication 3 ou 4, **caractérisé en ce que** l'hydrogénation du mélange hydrocarboné (KG1) est réalisée en présence d'un catalyseur, qui contient comme métal actif au moins un élément du 8ème au 10ème groupe du système périodique des éléments, en particulier du nickel ou du ruthénium.

6.  Procédé selon l'une quelconque des revendications 3 à 5, **caractérisé en ce que** le mélange hydrocarboné (KG1) contient du benzène, du méthylcyclopentane (MCP) et au moins un autre composé choisi parmi le cyclohexane, le cyclopentane, les oléfines ou les $C_5$-$C_8$-alcanes non cycliques.

7.  Procédé selon l'une quelconque des revendications 1 à 6, **caractérisé en ce que** dans l'étape a), le catalyseur est un liquide ionique acide, de préférence le liquide ionique acide contient comme cation un ion d'ammonium au moins partiellement alkylé ou un cation hétérocyclique et/ou comme anion un anion de chloroaluminium présentant la composition $Al_nCl_{(3n+1)}$ avec $1 < n < 2{,}5$.

8.  Procédé selon l'une quelconque des revendications 1 à 7, **caractérisé en ce qu'**au moins un composé, choisi parmi les $C_5$-alcanes linéaires ou ramifiés, le cyclopentane ou les $C_6$-alcanes linéaires ou ramifiés, est séparé par distillation avant l'étape a) du mélange hydrocarboné (KG1).

9.  Procédé selon l'une quelconque des revendications 1 à 8, comprenant en outre l'étape d), qui est réalisée avant l'étape a) et le cas échéant avant l'étape c), consistant à

    d) injecter le mélange hydrocarboné (KG1) dans un dispositif de distillation (D1),

    (KG1) contenant du méthylcyclopentane (MCP), au moins un alcane comprenant 7 atomes de carbone ou plus et le cas échéant un aromatique, au moins un alcane comprenant 7 atomes de carbone ou plus étant séparé de (KG1) dans l'étape (D1) avec obtention du mélange hydrocarboné (KG1b), et (KG1b) présentant une quantité réduite par rapport à (KG1) d'au moins un alcane comprenant 7 atomes de carbone ou plus et (KG1b) étant utilisé à la plage de (KG1) dans les étapes suivantes.

10. Procédé selon la revendication 9, **caractérisé en ce que** l'étape d) comprend les étapes partielles suivantes, consistant à :

    d1) injecter le mélange hydrocarboné (KG1) comprenant

    i) du benzène,
    ii) du MCP,
    iii) du diméthylpentane (DMP),
    iv) du cyclohexane et
    v) le cas échéant au moins un autre composé choisi parmi les oléfines ou les $C_5$-$C_8$-Alcanes

    dans le dispositif de distillation (D1),
    d2) séparer un flux (S3) contenant du DMP d'une sortie du dispositif de distillation (D1), la sortie se trouvant sous l'alimentation, de préférence au niveau du fond de (D1),

d3) séparer le mélange hydrocarboné (KG1b) contenant du benzène et/ou du MCP d'une sortie du dispositif de distillation (D1), la sortie se trouvant au-dessus de l'alimentation, de préférence au niveau de la tête de (D1).

11. Procédé selon la revendication 10, **caractérisé en ce que** le mélange hydrocarboné (KG1b) contient au moins 95%, de préférence au moins 98% de la quantité partielle, contenue dans le mélange hydrocarboné (KG1), constituée par le benzène et le MCP et/ou **en ce que** le mélange hydrocarboné (KG1b) contient au plus 0,1% en poids, de préférence au plus 0,02% en poids (par rapport à la quantité totale de benzène et de MCP dans (KG1b)) de DMP, de manière particulièrement préférée le mélange hydrocarboné (KG1b) contient au plus 0,015% en poids (par rapport à la quantité totale de benzène et de MCP dans (KG1b)) de 2,4-DMP.

12. Procédé selon l'une quelconque des revendications 9 à 11, **caractérisé en ce que** le flux (S3) est introduit dans un dispositif de distillation (D2), le cyclohexane étant séparé du DMP dans (D2), (D2) comprend de préférence une colonne de distillation extractive et/ou le flux enrichi en cyclohexane soutiré de (D2) contient de préférence au plus 0,1% en poids, de préférence au plus 0,02% en poids de DMP, de manière particulièrement préférée au plus 0,015% en poids de 2,4-DMP.

13. Procédé selon la revendication 12, **caractérisé en ce que** la séparation de cyclohexane/DMP comprend les étapes suivantes i) à iii) et le cas échéant l'étape iv), le dispositif de distillation (D2) étant formé par les trois éléments (D2-1) à (D2-3) :

i) une colonne de rectification (D2-1), dans laquelle la plus grande partie des substances lourdes présentant un point d'ébullition normal > 84°C (par rapport à la quantité dans l'alimentation vers (D2-1)) est séparée via le fond et la plus grande partie du cyclohexane et d'autres composés présentant un point d'ébullition normal de 79 à 84°C (par rapport à la quantité dans l'alimentation vers (D2-1)) est séparée via la tête,

ii) une colonne de distillation extractive (D2-2), dans laquelle le produit de tête de (D2-1) est rassemblé avec un adjuvant d'extraction et distillé de manière telle que la plus grande partie de l'adjuvant d'extraction et du cyclohexane est soutirée via le fond et la plus grande partie des autres composés contenus dans le produit de tête de (D2-1) présentant un point d'ébullition normal de 79 à 84°C est soutirée via la tête de (D2-2),

iii) une colonne de régénération (D2-3), dans laquelle la plus grande partie du cyclohexane contenu dans le flux de fond de (D2-2) est soutirée via la tête et la plus grande partie de l'adjuvant extraction contenu dans le flux de fond de (D2-2) est soutirée via le fond et

iv) le cas échéant un dispositif d'hydrogénation, dans lequel est guidé soit le flux (S3) soit le produit de tête de (D2-3).

14. Procédé selon la revendication 13, **caractérisé en ce que** le cyclohexane, qui provient du dispositif de distillation (D2), est rassemblé avec le cyclohexane qui a été préparé lors de l'isomérisation selon l'étape a) et le cas échéant lors de l'hydrogénation selon l'étape c).

15. Procédé selon l'une quelconque des revendications 1 à 14, **caractérisé en ce que** dans l'étape b), on isole du cyclohexane d'une pureté d'au moins 98% en poids, de préférence d'au moins 99,5% en poids, de manière particulièrement préférée d'au moins 99,9% en poids.

16. Procédé selon l'une quelconque des revendications 1 à 15, **caractérisé en ce que** le mélange hydrocarboné (KG2), contenant du cyclohexane, du MCP, le cas échéant des $C_5$-$C_6$-alcanes non cycliques et le cas échéant des composants à point d'ébullition plus élevé que le cyclohexane, est injecté dans une colonne de distillation (D4), la plus grande partie du MCP et le cas échéant des $C_5$-$C_6$-alcanes non cycliques contenus dans l'alimentation vers (D4) étant séparée de (D4) au niveau d'un site de prélèvement au-dessus de l'alimentation, de préférence via la tête et recyclée dans ou en amont de l'isomérisation selon l'étape a).

17. Procédé selon la revendication 16, **caractérisé en ce qu'**on soutire de la colonne de distillation (D4) du cyclohexane à une pureté d'au moins 98% en poids via le fond de (D4) ou via un soutirage latéral de (D4) situé sous l'alimentation, de préférence via un soutirage latéral sous forme vapeur de (D4).

18. Procédé selon la revendication 17, **caractérisé en ce que** le flux enrichi en cyclohexane soutiré via le fond de (D4) est introduit dans une colonne de distillation (D5), un flux (S5), contenant des composants à point d'ébullition plus élevé que le cyclohexane, étant séparé via le fond de (D5) et du cyclohexane d'une pureté d'au moins 98% en poids, de préférence d'au moins 99,5% en poids, de manière particulièrement préférée d'au moins 99,9% en poids, étant soutiré via un site de prélèvement au-dessus de l'alimentation vers (D5), de préférence via la tête.

**19.** Procédé selon la revendication 17, **caractérisé en ce qu'**un flux enrichi en cyclohexane est séparé via le soutirage latéral de la colonne de distillation (D4), le soutirage latéral se trouvant de préférence dans la partie d'épuisement de (D4) et/ou le flux enrichi en cyclohexane est guidé du soutirage latéral de (D4) dans un dispositif (D6) de préférence réalisé sous forme de colonne de distillation pour une nouvelle purification et on y obtient, via un site de prélèvement au-dessus de l'alimentation de (D6), de préférence via la tête, du cyclohexane d'une pureté d'au moins 98% en poids, de préférence d'au moins 99,5% en poids, de manière particulièrement préférée d'au moins 99,9% en poids.

**20.** Procédé selon l'une quelconque des revendications 16 à 19, **caractérisé en ce que** la colonne de distillation (D4) est réalisée sous forme de colonne à paroi de séparation, la paroi de séparation se trouve en partie sous le site d'alimentation, un site de soutirage se trouve au niveau de la paroi de séparation et un flux de cyclohexane de préférence liquide, présentant une pureté d'au moins 98% en poids, de préférence d'au moins 99,5% en poids, de manière particulièrement préférée d'au moins 99,9% en poids est prélevé via ce site de soutirage.

Fig. 1

Fig. 2

Fig. 3

Fig. 4

Fig. 5

Fig. 6

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20030109767 A
- EP 1403236 A
- US 20050082201 A
- WO 2010027987 A
- US 3311667 A
- EP 1995297 A
- EP 1992673 A

- US 2846485 A
- WO 2011069929 A
- WO 2011069957 A
- US 4053369 A
- US 4955468 A
- WO 0222528 A
- EP 1127601 B1

**In der Beschreibung aufgeführte Nicht-Patentliteratur**

- Kirk-Othmer Encyclopedia of Chemical Technology Published Online. 17. August 2001, vol. 8, 739 ff